# EUROPEAN PATENT SPECIFICATION

(11) **EP 4 617 660 B1**
(45) Date of publication and mention of the grant of the patent: **02.09.2026**
(21) Application number: 24163509.3
(22) Date of filing: 14.03.2024
(51) Int. Cl.: G01N 30/86, G01V 9/00, G01N 33/88, G01N 33/24, G01N 30/88

(54) **AN IMPROVED METHOD FOR DETERMINING THE RESERVOIR FLUID COMPOSITION**
VERBESSERTES VERFAHREN ZUR BESTIMMUNG DER RESERVOIRFLÜSSIGKEITSZUSAMMENSETZUNG
PROCÉDÉ AMÉLIORÉ POUR DÉTERMINER LA COMPOSITION DE FLUIDE DE RÉSERVOIR

(43) Date of publication of application: 17.09.2025
(73) Proprietor: Pietro Fiorentini S.p.A., 36057 Arcugnano (VI) (IT)
(72) Inventor: TEGELAAR, Erik, 2517JR 's-Gravenhage (NL); NEDERLOF, Peter, 2517JR 's-Gravenhage (NL); NARDI, Paolo, 2517JR 's-Gravenhage (NL); TAYLOR, Paul, 2517JR 's-Gravenhage (NL)
(74) Representative: Braidotti, Andrea

(56) References cited:
- US-A1- 2018 195 383

## Description

### FIELD OF INVENTION

The present invention relates to the technical field of oil and gas reservoirs. The method according to the invention is configured and intended to determine the reservoir fluid composition in terms of component quantities, in particular is configured and intended to determine the fluid composition of a petroleum reservoir in terms of hydrocarbon and/or non-hydrocarbon quantities.

### STATE OF THE ART - PRIOR ART

Petroleum present within a typical subsurface reservoir is a complex mixture of many thousands of different components, ranging from methane to macromolecules containing hundreds or thousands of carbon atoms. A robust understanding of the composition of the petroleum mixture in an accumulation is necessary to assess the viability of economic development, to estimate the number of producer and injector wells required for production, and to design adequate well completions and surface fluid handling facilities.

The known industry practice involves the collection of petroleum fluid samples from the reservoir using down-hole sampling devices, or at surface during well testing; usually dedicated metered oil and gas flow lines on a separator vessel are used. These fluid samples are then analyzed in a laboratory to determine their physical properties and molecular composition. The resulting compositional data (often mathematically combined) are used by reservoir and petroleum engineers as input to thermodynamic equations of state in numerical simulation software to predict fluid flow and phase behavior within the reservoir and in the production facilities.

It is not possible, both from analytical and computational perspectives, to characterize every individual molecular component within a petroleum fluid. Therefore, equations of state calculations use lumped pseudo-components as input. The thermodynamic properties of the pseudo-components are then mathematically adjusted ("tuned") to provide the best match between calculated and observed fluid properties. The known current analysis methods typically quantify hydrocarbon components and pseudo-components up to C36 with higher molecular weight material reported as a C36+ fraction.

A significant drawback of the known technology is the limited availability of physical fluid samples. The very high cost of down-hole sampling and well testing means that relatively few samples can be taken for PVT (pressure-volume-temperature) compositional analysis during the appraisal and development stages of a hydrocarbon accumulation. Moreover, in many cases samples of individual reservoirs can not be taken because of technical issues related to sampling equipment, low permeability of the rock matrix, or the complexity of the reservoir architecture prohibiting subsampling of each individual flow zone. This causes significant uncertainty in spatial reservoir fluid composition and consequently in the prediction of fluid phase behavior in reservoirs in which reservoir fluids are not at thermodynamic equilibrium. Such non-equilibrium conditions may occur naturally as a consequence of incomplete fluid mixing caused by multiple mechanisms, for example, heterogeneous reservoir rocks, active fluid migration or in-reservoir biodegradation.

A known solution is disclosed in US2018/195383.

### OBJECTS OF THE INVENTION

The object of the invention is to propose a method for determining the reservoir fluid composition, in particular for determining fluid composition in terms of hydrocarbons quantities, which overcomes, at least in part, the drawbacks of known solutions.

Another object of the invention is to provide a high-resolution fluid characterization at much reduced cost in comparison to the known solutions.

Another object of the invention is to propose a method that allows to reduce uncertainty of fluid composition and phase behavior.

Another object of the invention is to propose a method that allows to provide detailed compositional analysis data suitable for use in known industry standard workflows.

Another object of the invention is to propose a method that uses samples collected as part of routine drilling operations.

Another object of the invention is to propose a method that is of easy, quick and low-cost implementation.

Another purpose of the invention is to propose a method that is precise, reliable and accurate.

Another object of the invention is to propose a method that can be used to create easily and quickly a profile of the fluid composition at various depths, preferably so as to create a Reservoir Fluid Composition (RFC) Log.

Another object of the invention is to propose a method that can be used to create easily and quickly a detailed 3D representation of spatial fluid distributions including vertical and lateral fluid discontinuities and vertical and lateral fluid gradients.

Another object of the invention is to propose a method that has an alternative and/or improved characterization, both in constructive and functional terms, with respect to the traditional ones.

Another object of the invention is to propose a method that is alternative to the traditional ones.

Another object of the invention is to propose a method which meets high functional standards, and at the same time has an affordable cost, thus allowing the possibility of its utilization on a large scale.

Another object of the invention is to propose a method which meets high functional standards, and at the same time has an affordable cost, thus allowing the possibility of its utilization on a large scale.

### SUMMARY OF THE INVENTION

All these objects, both alone and in any combination thereof, and others which will result from the following description are achieved, according to the invention, with a method with the features of claim 1.

The present invention relates to a method for determining the fluid composition of a reservoir, in particular of a petroleum reservoir, by using at least one rock sample of the reservoir, said method comprising:
- a first analysis of first released gasses, that are released following to the application of a mechanical action, preferably a crushing action, on a first rock sub-sample of said rock sample, so as to determine the molecular quantities of a first hydrocarbons subgroup,
- a second analysis of second released gasses and/or a further analysis of further released gasses, that are released following to the application of a mechanical action, preferably a crushing action, on a second rock sub-sample of said rock sample, so as to determine the molecular quantities of a second hydrocarbons subgroup overlapping at least partially with said first hydrocarbons subgroup, and/or a further analysis of further released gasses, that are released following to the application of a mechanical action, preferably a crushing action, on a further rock sub-sample of said rock sample, so as to determine the molecular quantities of non-hydrocarbons and also of at least one hydrocarbon belonging to said first hydrocarbons subgroup,
- determining the molecular quantities of an hydrocarbons group comprising said first hydrocarbons subgroup and second hydrocarbons subgroup by using the molecular quantities so determined by means of said first analysis and said second analysis and/or determining the molecular quantities of non-hydrocarbons by using the molecular quantities so determined by means of said first analysis and by said further analysis.

The present invention relates to a method for determining the fluid composition of a reservoir, in particular of a petroleum reservoir, the method uses at least one rock sample of the reservoir for determining the molecular quantities of a hydrocarbons group having the carbon atoms within a given range, said method comprising:
- a first analysis of first released gasses, that are released following to the application of a mechanical action, preferably a crushing action, on a first rock sub-sample of said rock sample, so as to determine the molecular quantities of a first hydrocarbons subgroup having the carbon number within a first subrange of said given range,
- a second analysis of second released gasses, that are released following to the application of a mechanical action, preferably a crushing action, on a second rock sub-sample of said rock sample, so as to determine the molecular quantities of a second hydrocarbons subgroup having the carbon atoms within a second subrange of said range, wherein said second hydrocarbons subgroup overlaps partially with said first hydrocarbons subgroup,
- determining the molecular quantities of the hydrocarbons group by using the molecular quantities so determined of said first hydrocarbons subgroup and of said second hydrocarbons subgroup.

The present invention relates to a method for determining the fluid composition of a reservoir, in particular of a petroleum reservoir, the method uses at least one rock sample of the reservoir for determining the molecular quantities of hydrocarbons and also non-hydrocarbons, said method comprising:
- a first analysis of first released gasses, that are released following to the application of a mechanical action, preferably a crushing action, on a first rock sub-sample of said rock sample, so as to determine the molecular quantities of a first hydrocarbons subgroup,
- a further analysis of further released gasses, that are released following to the application of a mechanical action, preferably a crushing action, on a further rock sub-sample of said rock sample, so as to determine the molecular quantities of non-hydrocarbons and also of at least one hydrocarbon belonging to said first hydrocarbons subgroup,
- determining (64), by using the molecular quantities so determined by means of said first analysis (11) and by said further analysis (61), the molecular quantities of non-hydrocarbons and hydrocarbons belonging to the first hydrocarbons subgroup.

Preferably, the method provides the distribution of the molecular quantities of the hydrocarbons group subdivided by the number of carbon atoms, i.e. the pseudo-components of the hydrocarbons group. More preferably, according to the standard petroleum reservoir engineering practice, the pseudo-components comprise all components occurring between normal alkanes (e.g. pseudo-component P12 represents all material eluting from gas chromatographic analysis of an oil after nC11 up to, and including, nC12).

Said first hydrocarbons subgroup comprises C1 - C5 hydrocarbons (i.e. wherein the carbon atoms are in the first sub range 1 - 5). The first analysis provides the distribution of C1 - C5 alkane.

The pseudo-components of the first hydrocarbons subgroup C1 - C5 (i.e. the total composition of all hydrocarbons subdivided by carbon number in the first subrange 1 - 5) are derived directly by the integration of the molecular quantities obtained by said first analysis, i.e. obtained by the analysis of first released gasses, that are released following to the application of a mechanical action, preferably a crushing action, on a first rock sub-sample of said rock sample. The first analysis provides the distribution of C1 - C5 pseudo-components.

Said second hydrocarbons subgroup comprises C4 - C8 hydrocarbons (i.e. wherein the carbon atoms are in the second subrange 4 - 8). The second analysis provides the distribution of C4 - C8 alkane.

The pseudo-components of the second hydrocarbons subgroup C4 - C8 (i.e. the total composition of hydrocarbons subdivided by carbon number in the subrange C4 - C8) are derived directly by the integration of the molecular quantities obtained by said second analysis, i.e. obtained by the analysis of second released gasses, that are released following to the application of a mechanical action, preferably a crushing action, on a second rock sub-sample of said rock sample. The second analysis provides the distribution of C4 - C8 pseudo-components.

The method provides the combination of molecular quantities of the first hydrocarbons subgroup and of said second hydrocarbons subgroup by using the relative quantity of the hydrocarbons that are common/overlapped between the first hydrocarbons subgroup and the second hydrocarbons subgroup, so as to construct the relative quantities of all hydrocarbons of the first hydrocarbons subgroup and also of the second hydrocarbons subgroup.

The hydrocarbons that are common/overlapped between the first hydrocarbons subgroup and the second hydrocarbons subgroup comprise C4 and C5 hydrocarbons, and more preferably comprise butane, isobutane, pentane and isopentane. Ideally, the relative quantities of the hydrocarbons of the first and second subgroup, that together comprises the hydrocarbons in the range C1 to C8, are calculated by normalizing the quantity of the hydrocarbons C4 to C5 that are quantified in both analyses.

Said hydrocarbons group comprises the hydrocarbons in the range C1 - C8 (i.e. wherein the carbon atoms are in the range 1 - 8).

Advantageously, the method allows to reconstruct a distribution of the molecular quantities of the group of C1 - C8 hydrocarbons for the reservoir fluid; in particular, the group of hydrocarbons so determined has the carbon atoms within the given range of 1 - 8. More advantageously, the method allows to reconstruct a full distribution of C1 - C8 pseudo-components in the fluid reservoir, representing the total composition of the C1 - C8 hydrocarbons subdivided by carbon number. Preferably, the method provides the combination of the distribution of C1 - C5 alkane (obtained by the first analysis) and of the distribution of C4 - C8 alkane (obtained by the second analysis) using the molecular quantities C4 and C5 hydrocarbons as common/overlapping reference, so as to obtain a first distribution of the molecular quantities of C1 - C8 alkane. Advantageously, the method allows to reconstruct a full distribution of C1 - C8 alkane in the fluid reservoir.

Preferably, the method further comprises:
- a third analysis of oil extracted from a third rock sub-sample of said rock sample or of third released fluids, that are released by chemical treatment or thermal treatment or physical/mechanical action on a third rock sub-sample of said rock sample, so as to determine the molecular quantities of a third hydrocarbons subgroup having the carbon atoms within a third subrange of said given range,
- determining the molecular quantities of a wider hydrocarbons group by using the molecular quantities so determined of said first hydrocarbons subgroup, of said second hydrocarbons subgroup and of said third hydrocarbons subgroup.

Preferably, the fluids for the third analysis are obtained by solvent extraction of a third rock sub-sample of said rock sample.

Preferably, the method provides the distribution of the molecular quantities of the wider hydrocarbons group subdivided by the number of carbon atoms.

Preferably, said third hydrocarbons subgroup comprises C19 - C36 hydrocarbons (i.e. wherein the carbon atoms are in the third subrange 19 - 36).

Preferably, the third analysis provides the distribution of C19 - C36 n-alkane (i.e. linear alkanes or straight-chain alkanes).

Preferably, the pseudo-components of the third subgroup C19 - C36 (i.e. the total composition of hydrocarbons subdivided by carbon number in the third subrange 19 - 36) are derived directly from the molecular quantities obtained by said third analysis, i.e. obtained by the gas chromatography analysis of oil extracted from a third rock sub-sample that is released by chemical, physical or thermal extraction, preferably a solvent extraction, on a third rock sub-sample of said rock sample. Preferably, the third analysis provides the distribution of C19 - C36 pseudo-components.

Preferably, the pseudo-components of the third subgroup are calculated by considering the normal alkane of the third subgroup.

Preferably, the method further comprises the calculation step of the molecular quantities of a fourth hydrocarbons subgroup having the carbon atoms within a fourth subrange, said calculation step is based on the molecular quantities so determined of said third hydrocarbons subgroup.

More preferably, the molecular quantities of a further (fourth) hydrocarbons subgroup are calculated by extrapolation using the molecular quantities of said third hydrocarbons subgroup and assuming that in reservoir oil - as demonstrated in the published literature about petroleum fluids - the molar quantity of normal alkanes (or pseudo-components) with respect to their carbon atom numbers follow an exponential relationship.

Preferably, the method further comprises the calculation step of the molecular quantities of a fourth hydrocarbons subgroup having the carbon atoms within a fourth subrange, said calculation step is based on the molecular quantities so determined of said third and/or second hydrocarbons subgroup. Preferably, said fourth hydrocarbons subgroup comprises C8 - C18 alkane. Preferably, said fourth hydrocarbons subgroup comprises C8 - C18 pseudo-components.

Preferably, said calculated fourth hydrocarbons subgroup comprises the hydrocarbons having the carbon numbers within an intermediate subrange that is between the second subrange and the third subrange. Ideally, said calculated fourth hydrocarbons subgroup comprises C8 - C18 hydrocarbons (i.e. wherein the carbon atoms are in the intermediate subrange 8 - 18). Preferably, the pseudo-components of the fourth subgroup are calculated by the regression of the pseudo-component or normal alkane distribution of the third subgroup.

Preferably, the pseudo-components of the third and/or of the fourth subgroup C8 - C36 (i.e. the total composition of hydrocarbons subdivided by carbon number in the subranges 8 - 36) are derived directly by the integration of the molecular quantities obtained by said third analysis, i.e. obtained by the analysis of oil extracted from a third rock sub-sample of said rock sample or obtained by the analysis of third released hydrocarbons, that are released following extraction, preferably a solvent extraction, on a third rock sub-sample of said rock sample.

Preferably, in a possible embodiment, the pseudo-components in the fourth hydrocarbons subgroup C8 - C18 are calculated by extrapolation using the pseudo-components of the third subgroup C19 - C36 obtained by said third analysis and/or using the pseudo-components of the second subgroup C4 - C8 obtained by said second analysis.

Preferably, in a possible embodiment, the pseudo-components in the fourth hydrocarbons subgroup are obtained by a fourth analysis of fourth released hydrocarbons, that are released following to the application of a mechanical action, preferably a crushing action, on a fourth rock sub-sample of said rock sample, so as to determine the molecular quantities of the fourth hydrocarbons subgroup.

Preferably, the method reconstructs a further distribution of the hydrocarbons subdivided by carbon numbers belonging to the third and fourth subrange, wherein said further distribution is determined by using the molecular quantities of the third hydrocarbons subgroup obtained by said third analysis and by extrapolation of data resulting from the molecular quantities of said third and/or second hydrocarbons subgroup.

Preferably, said wider hydrocarbons group comprises the hydrocarbons in the range C1 - C36 (i.e. wherein the carbon atoms are in the range 1 - 36).

Preferably, the method provides:
- the combination of molecular quantities distributions of the first hydrocarbons subgroup and of said second hydrocarbons subgroup, so as to determine a first distribution of the hydrocarbons subdivided by carbon numbers belonging to the first and second subrange,
- the calculation of molecular quantities of the fourth hydrocarbons subgroup by using the molecular quantities of the third hydrocarbons subgroup, so as to determine a further distribution of the hydrocarbons subdivided by carbon numbers belonging to the third and the fourth subrange, wherein said further distribution having at least one carbon component that is common/overlapped with the first distribution,
- the further combination of the first distribution with the further distribution so determined so as to obtain a total distribution of the hydrocarbons subdivided by carbon numbers belonging to the full range.

Preferably, the first distribution is a distribution of C1 - C8 hydrocarbons, while the further distribution is a distribution of C8 - C36 hydrocarbons. Preferably, the common hydrocarbon - working as common/overlapping reference for the further combination of first distribution with the further distribution - is C8 hydrocarbon. Preferably, the total distribution is a distribution of C1 - C36 hydrocarbons.

Preferably, the merging of the first distribution with the further distribution so determined, so as to obtain a total wider distribution of the hydrocarbons subdivided by carbon numbers belonging to the full wider range, is performed:
- by rescaling the first distribution and the further distribution at the common carbon component, and
- by renormalizing, preferably to 100 mole%, the distribution so rescaled to obtain said total distribution of the hydrocarbons subdivided by carbon numbers belonging to the full range.

Advantageously, the method allows to reconstruct a full distribution of the molecular quantities of the wider group of C1 - C36 hydrocarbons for the reservoir fluid; in particular, the group of hydrocarbons so determined has the carbon atoms within the given range of 1 - 36. More advantageously, the method allows to reconstruct a full distribution of C1 - C36 pseudo-components in the fluid reservoir, representing the total composition of the C1 - C36 hydrocarbons subdivided by carbon number.

The reconstruction of the full C1-C36 hydrocarbon distribution may be obtained by using C1 - C36 pseudo-components and/or by using C1 - C36 normal alkane.

Preferably, the method comprises a further analysis of further released gasses, that are released following to the application of a mechanical action, preferably a crushing action, on a further rock sub-sample of said rock sample, so as to determine molecular quantities about non-hydrocarbon components. Advantageously, this allows to get information also on the non-hydrocarbon components present in the reservoir fluid, preferably carbon dioxide and hydrogen sulphide.

Preferably, the gasses so released are analysed so as to determine the molecular quantity of a further subgroup comprising non-hydrocarbon gases, preferably such as carbon dioxide and hydrogen sulphide, and also at least one of C1-C4 hydrocarbons.

Preferably, the gasses so released are analysed by way of gas chromatography with thermal conductivity detection.

Preferably, the abundance of non-hydrocarbon components carbon dioxide and hydrogen sulphide within the petroleum reservoir fluid can be calculated by using the relative quantity of at least one hydrocarbon that is common/overlapped between said further subgroup and the first hydrocarbons subgroup C1 - C5 or the full distribution of C1 - C36 hydrocarbons. Ideally, as common/overlapping hydrocarbon it is used the abundance of C1 hydrocarbon in the further subgroup and in full C1-C36 hydrocarbon distribution. More preferably, the resulting combined distribution containing the distribution of C1-C36 hydrocarbons, of carbon dioxide and of hydrogen sulphide is then renormalized to 100mole%.

Preferably, said at least one rock sample comprises rock samples collected by core and/or by drill cuttings. Ideally, said at least one rock sample comprises samples of drill cuttings, side wall cores and/or conventional core chips/samples.

Preferably, said at least one rock sample is a non-preserved (i.e. not retrieved to surface under confining pressure or refrigerated using dry ice or liquid nitrogen) rock sample of the reservoir.

Preferably, said at least one rock sample has a known subsurface origin.

Preferably, the first rock sub-sample, the second rock sub-sample, the third rock sub-sample, the fourth rock sub-sample and/or the further rock sub-sample (or other sub-samples that may be used for analysis) are obtained from the same rock sample or by rock samples having the same origin. More preferably, the rock sample/s - ideally after disaggregation - is/are divided into four or more sub-samples.

Preferably, the first released gasses, the second released gasses and the further released gases are released by applying a mechanical crushing to the corresponding rock subsamples of the rock sample. Preferably, in a possible embodiment, also the third released hydrocarbons and the fourth released components may be released by applying a mechanical crushing to the corresponding rock subsamples of the rock sample or by way of solvent extraction, vacuum extraction, centrifuge extraction or thermal extraction.

For example, the rock sub-samples obtained from drill cuttings, side wall core chips and/or conventional core chips are placed into a sealed vessel and are mechanically crushed so as to liberate the entrained gas, thus obtaining said released gasses.

Preferably, the rock samples are hand-picked to differentiate between different lithofacies.

Preferably, said first analysis is performed via gas chromatography. More preferably, said first analysis is performed via gas chromatography with a flame ionization detector (GC-FID). Ideally, said first analysis is performed via gas chromatography to assess molecular quantity of the C1 - C5 hydrocarbons, and in particular of the hydrocarbons comprising methane, ethane, propane, isobutane, normal butane, neopentane, isopentane, normal pentane.

Preferably, said second analysis is performed via gas chromatography. More preferably, said second analysis is performed via gas chromatography with a flame ionization detector (GC-FID). Ideally, said second analysis is performed via gas chromatography to assess molecular quantity of the C4 - C8 hydrocarbons, and in particular of all isomers of the alkanes, cycloalkanes and aromatic compounds in the range C4 - C8.

Preferably, said second analysis is performed with analytical conditions that are different in respect to the analytical condition of the first analysis. Ideally, the second analysis is performed with modified temperature and chromatographic conditions in respect to the first analysis.

Preferably, the oil for the third analysis is extracted from a third rock sub-sample by solvent extraction. Ideally, the oil for the third analysis is extracted from a third rock sub-sample by using a non-polar organic solvent.

Preferably, the oil for the third analysis is extracted from a third rock sub-sample by thermal extraction.

Preferably, said third analysis is performed via gas or liquid chromatography. More preferably, said third analysis is performed via chromatography with a flame ionization detector.

Ideally, said third analysis is performed via gas chromatography to assess molecular quantity of the C19 - C36 hydrocarbons.

Preferably, said third analysis is performed via gas chromatography with a flame ionization detector (GC-FID). Ideally, said third analysis is performed via gas chromatography to assess molecular quantity of the C19 - C36 hydrocarbons, and in particular of all isomers of the alkanes, cycloalkanes and aromatic compounds in the range C19 - C36.

Preferably, when said fourth analysis for determining the fourth sub-group of hydrocarbons comprises the analysis of further released gasses, said fourth analysis is performed via gas chromatography. More preferably, said fourth analysis is performed via gas chromatography with a flame ionization detector (GC-FID). Ideally, said further analysis is performed via gas chromatography to assess molecular quantity of the C8 - C18 hydrocarbons, and in particular of all isomers of the alkanes, cycloalkanes and aromatic compounds in the range C8 - C18.

Preferably, said further analysis for determining non-hydrocarbons components is performed via gas chromatography. Preferably, said further analysis is performed with analytical conditions that are different in respect to the analytical condition of the first and second analysis. Ideally, the further analysis is performed with modified temperature and chromatographic conditions in respect to the first and second analysis.

Preferably, the analyses of the method are performed for each rock sample of a plurality of rock samples collected at different known depths of the reservoir fluid, so as to generate a profile of the fluid composition of the reservoir for each different known depth.

More preferably, the method comprises the analyses for each rock sample coming from a plurality of different known depths/origin in the reservoir.

Preferably, said method is characterized in that the molecular quantities of the hydrocarbons group, more preferably the distribution of the molecular quantities of the wider hydrocarbons group subdivided by the number of carbon atoms, is associated to the reservoir origin/depth of the corresponding rock samples used for the analyses, so as to define/build a reference profile (for example organized as a look-up table, a log or other organized dataset wherein to each input is associated the related output) comprising for each reservoir origin/depth of the rock sample used for the analyses the corresponding molecular quantities of the wider hydrocarbons group, more preferably the distribution of the molecular quantities of C1-C36 hydrocarbons subdivided by the number of carbon atoms. More preferably, the C1-C36 hydrocarbon distribution is supplemented with relative molecular abundance of non-hydrocarbon species, ideally carbon dioxide and hydrogen sulphide.

Ideally, the analyses of the method are performed for each rock sample coming from a plurality of different known depths/origin in the reservoir so as to determine for each rock sample the corresponding molecular quantities of the hydrocarbons and possibly also of non-hydrocarbons group, more preferably to determine the distribution of the molecular quantities of C1 - C36 hydrocarbons subdivided by the number of carbon atoms, and possibly also of carbon dioxide and hydrogen sulphide, thus generating corresponding logs of reservoir fluid composition at a given known depth/origin.

Preferably, the method provides the combination of a plurality of reservoir fluid composition logs to generate a 3D representation of spatial fluid distributions in the reservoir, ideally comprising also vertical and lateral fluid discontinuities and vertical and lateral fluid gradients.

The skilled person will appreciate that all preferred or optional features of the invention described with reference to only some aspects or embodiments of the invention may be applied to all aspects of the invention.

It will be appreciated that optional features applicable to one aspect of the invention can be used in any combination, and in any number. Moreover, they can also be used with any of the other aspects of the invention in any combination and in any number. This includes, but is not limited to, the dependent claims from any claim being used as dependent claims for any other claim in the claims of this application.

### DESCRIPTION OF THE FIGURES

The present invention is hereinafter further clarified in some of its preferred embodiments shown for purely exemplifying and non-limiting purposes with reference to the accompanying drawings, in which:
- Figure 1A: shows a diagram of the steps in the method according to the invention,
- Figure 1B: shows a diagram of the steps in a further embodiment of the method according to the invention,
- Figure 1C: shows a diagram of the steps of another further embodiment of the method according to the invention,
- Figure 2A: shows an example of gas chromatograms of a first sub-group of hydrocarbons, and in particular of C1 - C5 hydrocarbons, in first gasses released from a first rock sub-sample,
- Figure 2B: shows an example of gas chromatograms of a second sub-group of hydrocarbons, and in particular of C4 - C8 hydrocarbons, in second gasses released from a second rock sub-sample,
- Figure 2C: shows the gas chromatograms of Fig. 2A and Fig. 2B highlighting the area of their overlap that is used to compare and combine data from the two respective analyses,
- Figure 3: shows the normalized molecular composition (i.e. the first distribution) resulting from the combination of the analysis of Fig. 2A and Fig. 2B,
- Figure 4: shows an example of gas chromatograms of a third sub-group of hydrocarbons in an oil extracted from a rock sample,
- Figure 5: shows a reconstruction of the normalized molecular composition of a hydrocarbons group, and in particular of the group containing a plurality or all C1 - C36 hydrocarbons, by using the normal alkane data resulting from the analysis of the first, second and third sub-groups of hydrocarbons,
- Figure 6: shows a reconstruction of the normalized molecular composition of a hydrocarbons group, and in particular of the group containing a plurality or all C1 - C36 hydrocarbons, by using the pseudo-component (PC) data resulting from the analysis of the first, second and third sub-groups of hydrocarbons,
- Figure 7: shows an example of a gas chromatogram of the further sub-group comprising hydrocarbons and non-hydrocarbons, and in particular of C1 - C5 hydrocarbons, carbon dioxide and hydrogen sulphide as resulting from the further analysis of the gasses released from a further rock sub-sample,

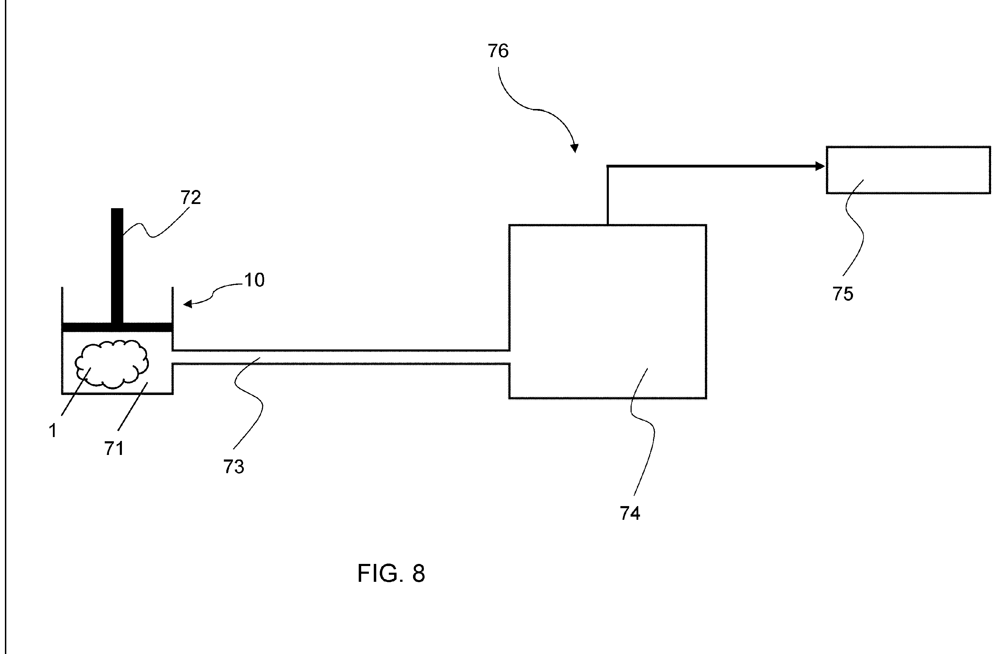

- Figure 8: shows a general schematic view of an apparatus used to release gas from rock subsamples.

### DETAILED DESCRIPTION OF THE INVENTION AND OF ITS PREFERRED AND EXEMPLARY EMBODIMENTS

The method according to the invention is used for determining the fluid composition of a reservoir, in particular of a petroleum reservoir, and uses at least one rock sample of the reservoir for determining the molecular quantities of a group of hydrocarbons group having the carbon atoms within a given range.

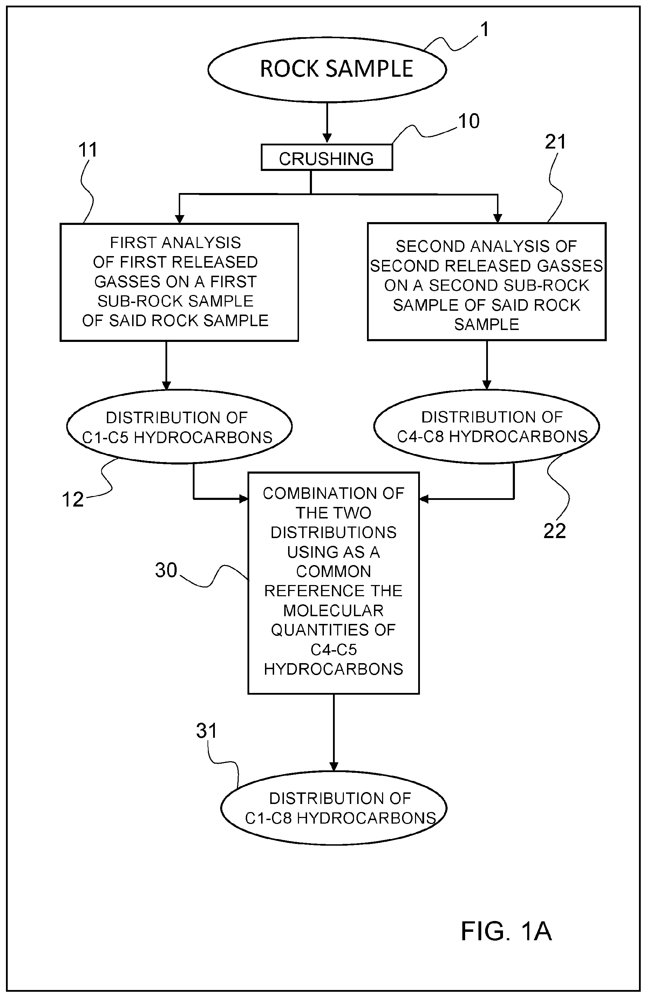

A first embodiment of the method according to the invention is shown in Fig. 1A. In particular, this method comprises:
- a first analysis 11 of first released gasses, that are released following to the application of a mechanical action, preferably a crushing action 10, on a first rock sub-sample of said rock sample 1, to determine the molecular quantities of a first hydrocarbons subgroup having the carbon number within a first subrange of said given range, in particular to determine/obtain a distribution 12 of the molecular quantities of said first hydrocarbons subgroup,
- a second analysis 21 of second released gasses, that are released following to the application of a mechanical action, preferably a crushing action 10, on a second rock sub-sample of said rock sample 1, so as to determine the molecular quantities of a second hydrocarbons subgroup having the carbon atoms within a second subrange of said range, in particular to determine/obtain a distribution 22 of the molecular quantities of said second hydrocarbons subgroup overlapping partially with said first hydrocarbons subgroup,
- determining the molecular quantities of the hydrocarbons group by using the molecular quantities so determined of said first hydrocarbons subgroup and of said second hydrocarbons subgroup; in particular, said step provides the combination 30 of the distribution 12 of the molecular quantities of said first hydrocarbons subgroup with the the distribution 22 of the molecular quantities of said second hydrocarbons subgroup so as to determine a first distribution 31 of the molecular quantities of the hydrocarbons group comprising the first subgroup and the second subgroup.

Preferably, said first hydrocarbons subgroup comprises C1 - C5 hydrocarbons. Preferably, said second hydrocarbons subgroup comprises C4 - C8 hydrocarbons. Preferably, said hydrocarbons group comprises C1 - C8 hydrocarbons. Preferably, the hydrocarbons that are common/overlapped between the first hydrocarbons subgroup and the second hydrocarbons subgroup comprise C4 and C5 hydrocarbons.

Advantageously, the present invention provides the combination 30 of the C1-C5 hydrocarbons distribution 12, obtained by the first analysis 11, with the C4-C8 hydrocarbons distribution 22, obtained by the second analysis 21, to determine the C1 - C8 hydrocarbons distribution 31.

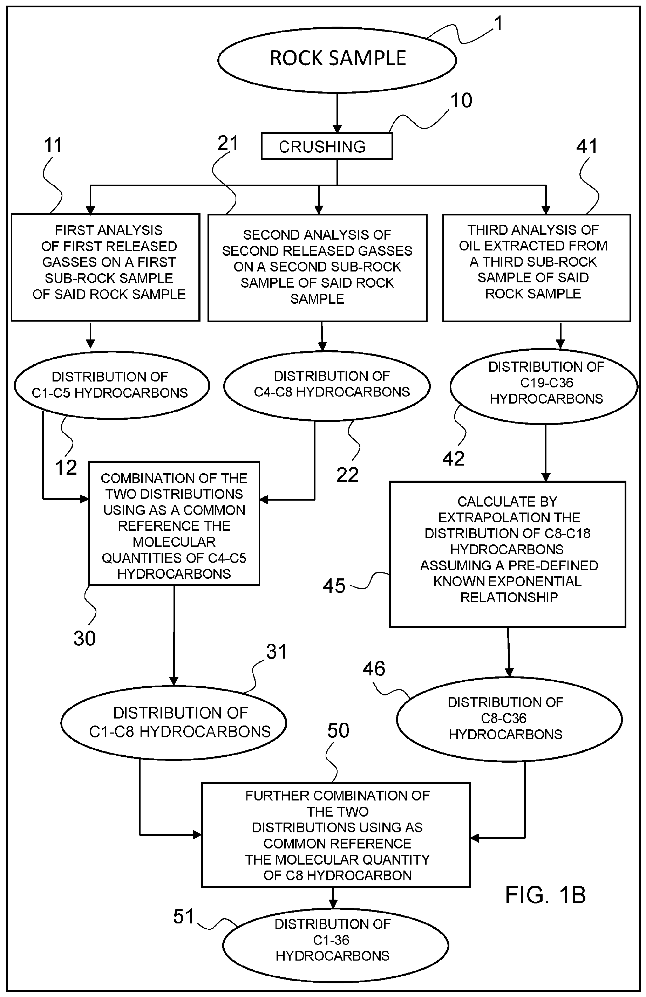

A further preferred embodiment of the method according to the invention is shown in Fig. 1B. In particular, in this further preferred embodiment, in addition to the steps of Fig. 1A and to the combination 30 of molecular quantities distribution of the first hydrocarbons subgroup and of said second hydrocarbons subgroup, so as to determine a first distribution 31 of the hydrocarbons subdivided by carbon numbers belonging to the first and second subrange, the method also comprises:
- a third analysis 41 of oil extracted from a third rock sub-sample of said rock sample, to determine the molecular quantities of a third hydrocarbons subgroup having the carbon atoms within a third subrange of said given range, in particular to determine the distribution 42 of the molecular quantities of said third hydrocarbons subgroup,
- the calculation 45 of molecular quantities of the fourth hydrocarbons subgroup by using the molecular quantities of the third hydrocarbons subgroup, to determine a further distribution 46 of the hydrocarbons subdivided by carbon numbers belonging to the third and the fourth subrange, wherein said further distribution 46 having at least one carbon component that is common/overlapped with the first distribution 31,
- the further combination 50 of the first distribution 31 with the further distribution 46 so determined so as to obtain a total distribution 51 of the hydrocarbons subdivided by carbon numbers belonging to the wider full range.

Ideally, said total distribution 51 is then normalized.

Preferably, said third hydrocarbons subgroup comprises C19 - C36 hydrocarbons. Preferably, said fourth hydrocarbons subgroup comprises C8 - C18 hydrocarbons. Preferably, said first distribution 31 of hydrocarbons comprises the distribution of molecular quantities of C1 - C8 hydrocarbons. Preferably, said further distribution 46 of hydrocarbons comprises the distribution of molecular quantities of C8 - C36 hydrocarbons. Preferably, C8 hydrocarbon is the hydrocarbon that is common/overlapped between the first hydrocarbons distribution 31 and the further hydrocarbons distribution 46. Preferably, said wider/total distribution 51 of hydrocarbons comprises the distribution of molecular quantities of C1 - C36 hydrocarbons.

Advantageously, the present invention comprises a combination of the C1-C5 hydrocarbons distribution 12, obtained by the first analysis 11, with the C4-C8 hydrocarbons distribution 22, obtained by the second analysis 21, so as to determine the C1 - C8 hydrocarbons distribution 31, a third analysis 41 to determine the C19 - C36 hydrocarbons distribution and a calculation 45 by extrapolation, using the C19 - C36 hydrocarbons distribution 42 so as to determine a further distribution 46 of C8 - C36 hydrocarbons, a further combination 50 of the C1 - C8 hydrocarbons distribution 31 with the C8 - C36 hydrocarbons distribution 46 to provide a normalized C1-C36 hydrocarbons wider/full distribution 51 for a given rock sample.

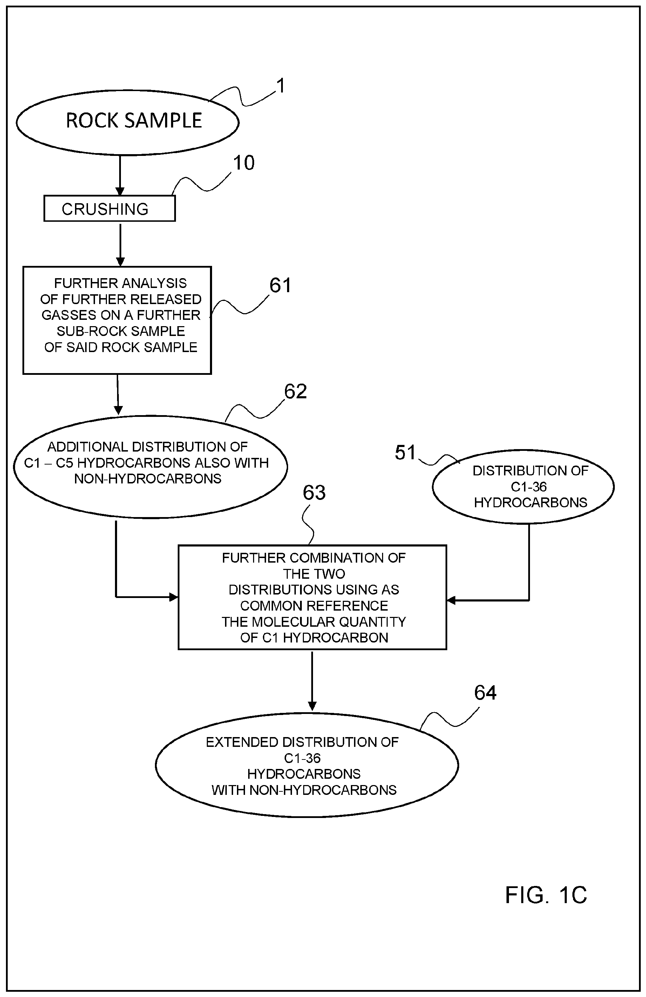

A further preferred embodiment of the method according to the invention is shown in Fig. 1C. In particular, in this further preferred embodiment, in addition to the steps of Fig. 1A and Fig. 1B and to the combination 51 of molecular quantities distribution of the C1-C36 hydrocarbons, the method also comprises:
- a further analysis 61 of further released gasses, that are released following to the application of a mechanical action, preferably a crushing action 10, on a further rock sub-sample of said rock sample, to determine the molecular quantities of a further subgroup comprising hydrocarbons and also non-hydrocarbons, in particular to determine an additional distribution 62 of the molecular quantities of at least one of C1-C5 hydrocarbons and also of carbon dioxide and hydrogen sulphide as non-hydrocarbons,
- determining a combined distribution 64 of C1-C36 hydrocarbons with carbon dioxide and hydrogen sulphide as non-hydrocarbons by using the total distribution 51 of C1 - C36 and the additional distribution 62 of the molecular quantities of C1-C5 hydrocarbons with carbon dioxide and hydrogen sulphide as non-hydrocarbons; in particular, said step provides the combination 63 of the total distribution 51 of the molecular quantities of C1 - C36 hydrocarbons with the additional distribution 62 of the molecular quantities of C1-C5 hydrocarbons with carbon dioxide and hydrogen sulphide as non-hydrocarbons by using the relative abundance of C1 hydrocarbon as common reference, so as to obtain the combined distribution 64 of C1-C36 hydrocarbons with carbon dioxide and hydrogen sulphide as non-hydrocarbons.

Ideally, said combined distribution 64 is then normalized.

More in detail, in a possible embodiment (see figure 1B), the method comprises the following steps of:
a) mechanically crushing (step 10) a first sub-sample of a rock sample of the reservoir to release the first gasses (i.e. to liberate first occluded gasses),
b) performing the first analysis (11) by analyzing the first released gasses, preferably via gas chromatography with flame ionization detector, to determine the molecular quantities of C1-C5 hydrocarbons (comprising methane, ethane, propane, isobutane, normal butane, neopentane, isopentane, normal pentane), in particular to obtain the distribution 12 of molecular quantities of C1-C5 hydrocarbons,
c) repeating step a) on a second sub-sample of a rock sample of the reservoir to release the second gasses (i.e. to liberate second occluded gasses) and performing the second analysis (21) of said second released gasses using different temperature and chromatographic conditions in respect to the first analysis (11) in order to determine the molecular quantities of all isomers of the alkanes, cycloalkanes and aromatic compounds of C4 - C8 hydrocarbons, in particular to obtain the distribution 22 of molecular quantities of C4 - C8 hydrocarbons,
d) combining (step 30) the molecular distributions obtained in steps b) and c) using as common/overlapping reference the relative quantities of C4 - C5 hydrocarbons to construct a first distribution 31 of relative molecular quantities of C1 - C8 hydrocarbons,
e) extracting oil from a third sub-sample of a rock sample of the reservoir, preferably using a non-polar organic solvent, and analyzing (step 41) the oil so extracted, preferably by gas chromatography with flame ionization detector,
f) integrating all normal alkanes or pseudo-components in the oil chromatogram obtained in step e),
g) determining the distribution 42 of the molecular quantities of C19 - C36 normal alkanes or pseudo-components (or of components that are not affected by evaporation),
h) calculating (step 45) an exponential curve fitting through the C19 - C36 normal alkanes or pseudo-components or through normal alkanes/pseudo-components that are not affected by evaporation as determined in step g),
i) extrapolating the molecular quantities of normal alkanes or pseudo-components C8 - C18 using the exponential equation obtained in step h) so as to determine a further distribution 46 with the molecular quantities of C8 - C36 hydrocarbons,
j) further combining (step 50) the further distribution of C8 - C36 hydrocarbons obtained in step i) and the first distribution of C1-C8 hydrocarbons obtained in step d) by using the quantity of C8 hydrocarbon that is a common reference for both distributions, so as to obtain a full distribution 51 of C1-C36 hydrocarbons,
k) re-normalizing to 100% the full distribution 51 of C1-C36 hydrocarbons of step j),

Preferably, in a further possible embodiment (see figure 1C), the method comprises also the following steps of:
l) repeating step a) on a further sub-sample of a rock sample to release further gasses (i.e. to liberate further occluded gasses) and performing the further analysis 61 of said further released gasses using different temperature and chromatographic conditions in respect to the first analysis 11 in order to determine the molecular quantities of C1, carbon dioxide and hydrogen sulphide,
m) combining (step 63) the molecular distributions obtained in steps k) and l) using as common/overlapping reference the relative quantities of C1 hydrocarbon (methane) to construct a reservoir fluid combined distribution 62 comprising the distribution 51 of C1-C36 hydrocarbons and also of carbon dioxide and hydrogen sulphide,
n) re-normalizing to 100% the combined distribution 62 of step m).

Preferably, in a possible embodiment not shown in the figure, the method may comprise only and specifically the first analysis 11 to determine the molecular quantities of a first hydrocarbons subgroup (for example of C1-C5 hydrocarbons) and said further analysis 61 so as to determine the molecular quantities of at least one hydrocarbon of said first hydrocarbons subgroup (for example of C1) and also of non-hydrocarbons, for example carbon dioxide and/or hydrogen sulphide, in order to determine then the molecular distributions of the hydrocarbons of said first subgroup and also of non-hydrocarbons, for example carbon dioxide and/or hydrogen sulphide. In particular, such determination step is made by combining the molecular quantities, obtained by the first analysis 11 and by said further analysis 61, using as common/overlapping reference the quantity - obtained both by the first analysis 11 and by said further analysis 61 - of said at least one hydrocarbon of said first hydrocarbons subgroup (for example of C1).

Preferably, rock samples from the zones of interest are collected by sampling core plugs or rock chips from conventional cores, side wall cores or drill cuttings. Preferably, cuttings should be gently washed to remove any drilling mud residues. Preferably, samples are disaggregated to 1-3 mm size before homogenization and splitting into three sub-samples.

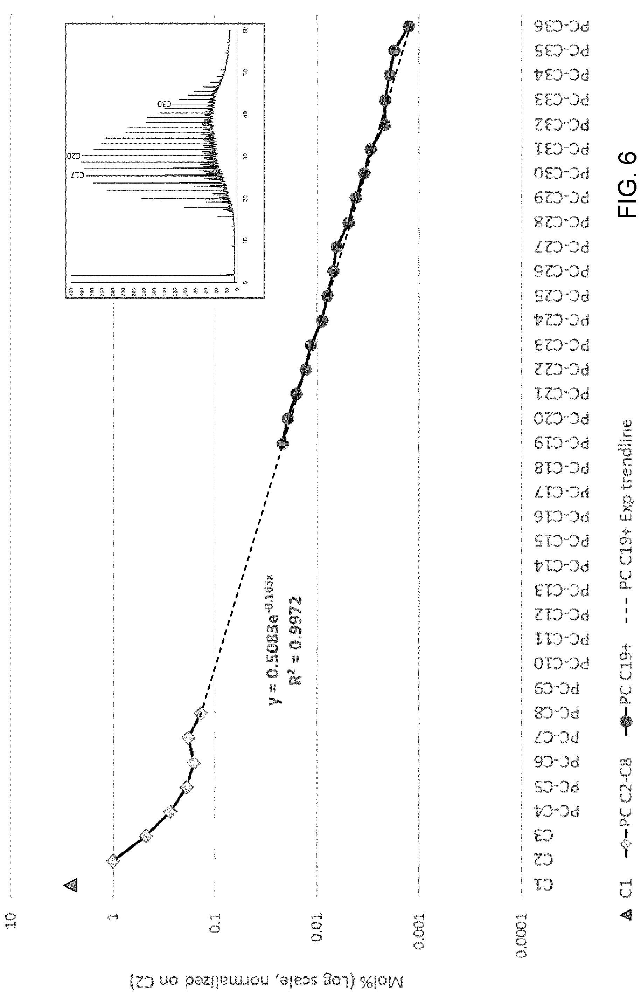

The released gases from the corresponding rock subsamples (in particular for the first and/or second subsamples) may be collected as follows. A sample of washed and dried rock 1 is placed into a gas tight receptacle 71 provided with a mechanical crushing device 72. The receptacle 71 is connected by means of a flow line 73 to an analytical instrument 76 comprising a gas chromatograph 74 with a detector 75 (typically flame ionization detector or thermal conductivity detector) for gas composition and of liberated gas components. The rock sample 1 is crushed to release entrained gas, which is then transferred to the analytical instrument 76 via the flow line 73 (see Figure 6).

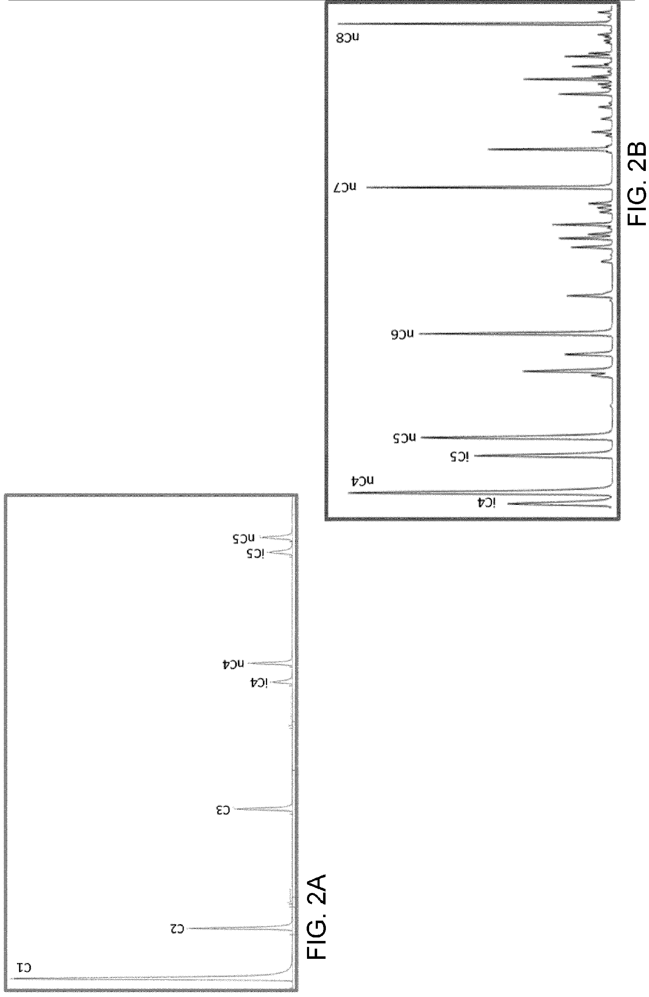

The first analysis 11 of a first sub-sample can achieve quantitative identification of hydrocarbons in the range C1 to C5 (see fig. 2A). The second analysis 21 is performed as the first analysis but with modified temperature conditions on the second sub-sample and this allows the quantitative identification of hydrocarbons in the range C4 to C8 (see fig. 2B).

For example, the first analysis 11 is performed using an Agilent 6890N gas chromatograph (Agilent Technologies, Santa Clara, US) equipped with a 30m 0.32mm Gaspro column (also from Agilent) and a flame ionization detector. Gases released from the crusher apparatus are trapped in liquid nitrogen before being released into a flow of helium onto the GC column and are separated using an oven temperature program that ramps from 50C to 260C, while the second analysis 21 is performed using the same apparatus but with a modified chromatographic column (Agilent, DB-1) and oven temperature program, that ramps from 30C to 250C.

For example, the further analysis 61 for determining the molecular quantities of C1 - C5 hydrocarbons (or at least of C1 hydrocarbon) with non-hydrocarbons is performed using same apparatus but with a modified chromatographic column (for example Agilent, Porabond Q) and an oven temperature program, that ramps from about 35°C to 250°C, with thermal conductivity detector.

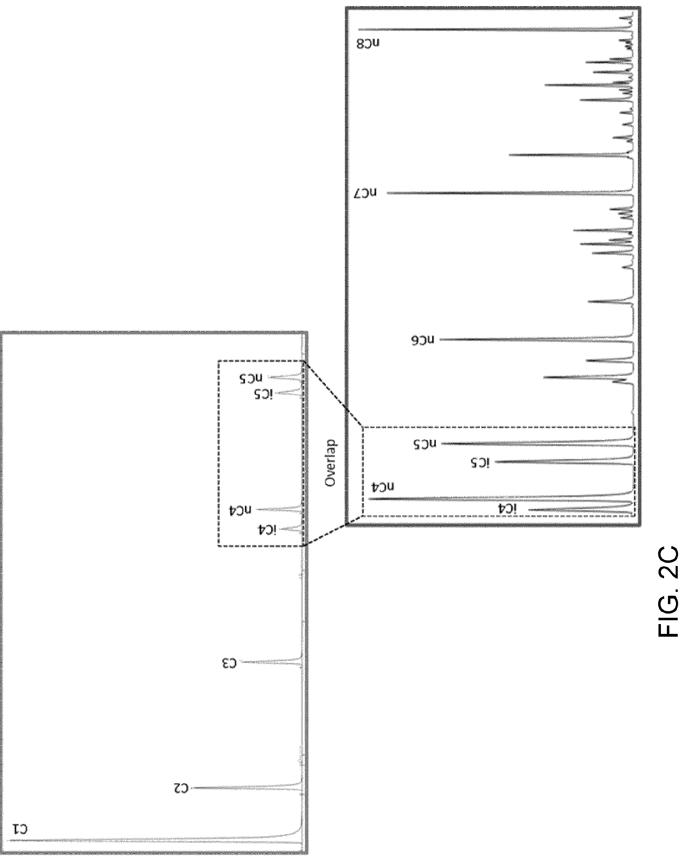

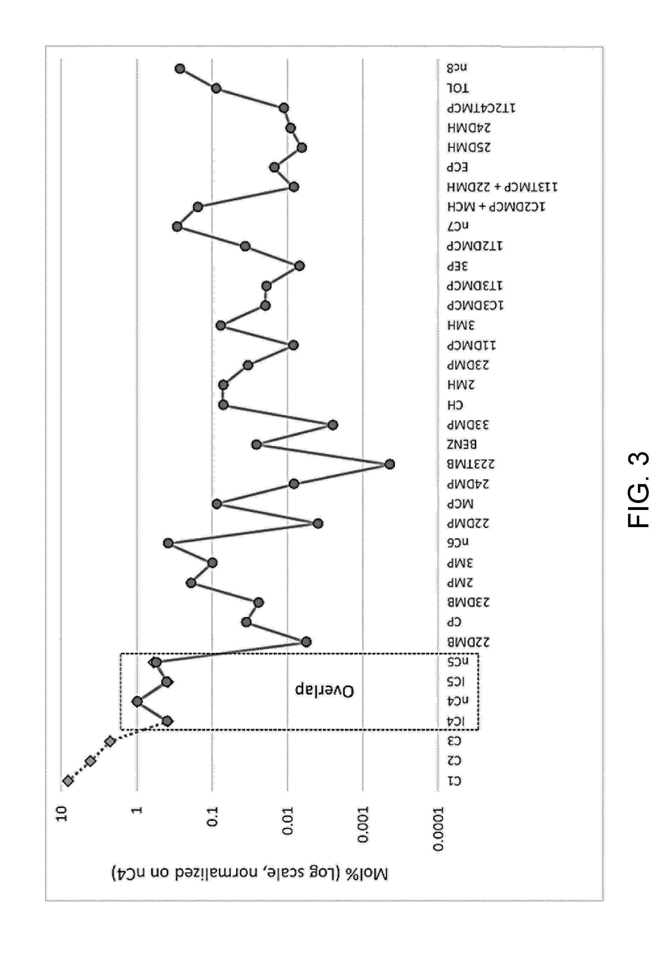

The relative quantities of the separated hydrocarbons in the range C1 to C8 are calculated by normalizing the quantity of components C4 to C5 that are common in both analyses (see fig. 2C). This allows to determine the pseudo-components of the C1-C8 hydrocarbons, that is the total composition of hydrocarbons subdivided by carbon number 1 - 8 (see fig 3).

Preferably, the third sub-sample of rock for the third analysis 41 is soaked in a non-polar organic solvent, such as cyclohexane, to dissolve crude oil present within the sample. Removal of oil can be enhanced by standard extraction methods involving heating or sonication. An aliquot of the solvent is removed and (if necessary) suspended sediment removed by filtration. The supernatant liquid may be injected directly into a gas chromatograph for analysis of the extracted oil material, or if oil yield is too low to achieve a good response then excess solvent is removed by evaporation prior to GC analysis.

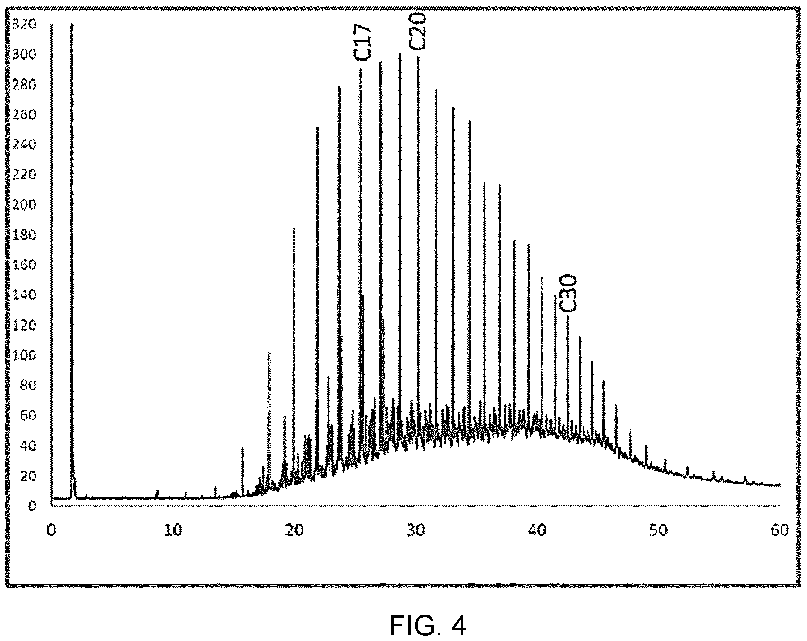

The resulting gas chromatogram of the third analysis (see fig. 4) corresponds to the output of oil compositional analysis carried out as part of a standard reservoir fluid analysis program, with the exception that some of the more volatile components of the oil are lost to evaporation. In particular, the evaporation of volatile components occurs during sample transport to surface, during storage and during analysis and so is expected to occur in all samples to some extent in components with less than about fifteen carbon atoms.

Preferably, the output of the oil gas chromatogram of the third analysis (see fig. 4) is integrated to tabulate the areas of all peaks corresponding the homologous series of normal alkanes and also to determine abundance of pseudo-components. Subsequently, peak areas of normal alkanes and of pseudo-components are converted to molar fractions using estimated molecular weights according to standard engineering practice. Therefore, this allows the quantitative identification of hydrocarbons in the range C19 to C36.

Preferably, the oil for the third analysis 41 may be extracted from a third rock sub-sample by solvent extraction. Ideally, the oil for the third analysis 41 may be extracted from a third rock sub-sample by using a non-polar organic solvent. Preferably, the oil for the third analysis 41 may be extracted from a third rock sub-sample by thermal extraction. Preferably, said third analysis 41 may be performed via gas or liquid chromatography. More preferably, said third analysis 41 may be performed via chromatography with a flame ionization detector.

The published literature in the technical field of analysis of petroleum reservoir fluids - see in particular "Compositional regularities common to petroleum reservoir fluids and pyrolysates of asphaltenes and kerogens" of Keith F.M. Thompson, 2002 in "Organic Geochemistry", Volume 33, Issue 7, July 2002, pages 829-841 and "Catagenesis and composition of petroleum: Origin of n-alkanes and isoalkanes in petroleum crudes" of Y.V. Kissin, 1987 in "Geochimica et Cosmochimica Acta", Volume 51, Issue 9, September 1987, pages 2445-2458" - has shown that the molar quantity of normal alkanes in unaltered crude oil displays an exponential relationship to the carbon number of the molecule above C10 and this relationship holds from C6. In particular, according to a pre-defined model derived from the published literature, it results that that the molar concentrations of n-alkanes in reservoir fluids decrease exponentially with increasing carbon number. More in detail, two series, C2-n-C5, and n-C6+, are separated by a slope break, the former being steeper; moreover, the pseudo-components, representing the total composition subdivided by carbon number, also decrease in concentration exponentially beyond C10.

Hence, by using the normal alkane or pseudo-component distribution C19 - C36 and by using a predefining model (that is based on the above-mentioned published literature in the technical field of petroleum reservoir fluids analysis) according to which the molar quantities of normal alkanes and pseudo-components in unaltered crude oil displays an exponential relationship to the carbon number of the molecule above C6, the molecular quantities of at least C8 - C18 normal alkanes are calculated by extrapolation. More in detail, a curve fit through normal alkane distribution C19 - C36 - i.e. normal alkanes that are not affected by evaporation - allows to calculate then by extrapolation the molar quantity of all normal alkanes C8 - C18. Therefore, the further distribution 46 of molar quantities of C8 - C36 normal alkane is obtained. This allows to determine the pseudo-components of the C8-C36 hydrocarbons, that is the total composition of hydrocarbons subdivided by carbon number 8 - 36.

Advantageously, the published literature in the technical field of analysis of petroleum reservoir fluids - see in particular "Compositional regularities common to petroleum reservoir fluids and pyrolysates of asphaltenes and kerogens" of Keith F.M. Thompson, 2002 in "Organic Geochemistry", Volume 33, Issue 7, July 2002, pages 829-841 - also shows that there is a direct relationship between the regression of normal alkane data and lumped pseudo-components data in the same oil, which means that n-alkanes may, in a first approximation, act as a proxy for true pseudo-components data.

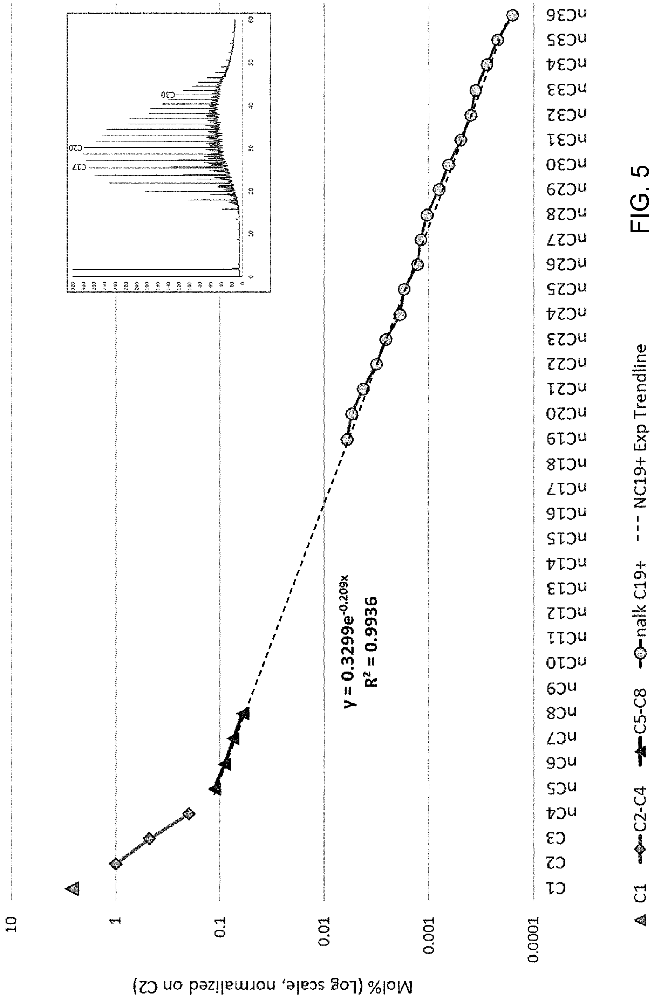

Once the molar quantities of C8 - C36 normal alkane has been determined, the full C1-C36 hydrocarbon composition is determined by rescaling the C1-C8 distribution and the C8-C36 distribution at the common/overlapping reference defined by of C8, and renormalizing to 100 mole% so as to generate the C1-C36 distribution (see fig. 5). The same procedure can be carried out using pseudo-component data (see fig. 6).

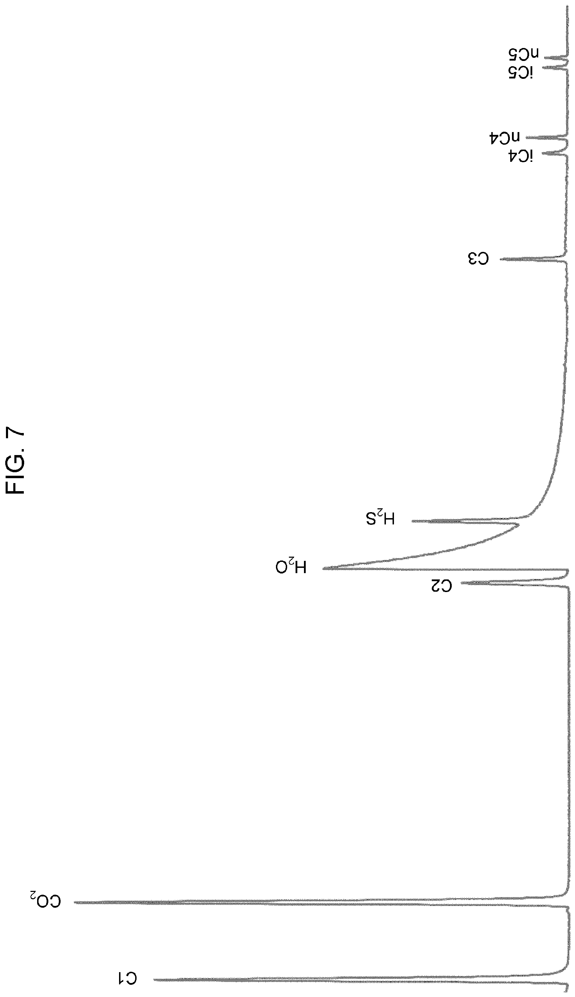

Advantageously, the method may be extended to further characterize the abundance of non-hydrocarbon gases carbon dioxide and hydrogen sulphide in the reservoir fluid, by way of analysis of a further rock sub-sample. Gases from said further sub-sample are released by crushing and analysed using gas chromatography with thermal conductivity detection to quantify the abundance of C1-C5 hydrocarbons and non-hydrocarbons carbon dioxide and hydrogen sulphide (see figure 7). The relative abundance of the non-hydrocarbon components within the total reservoir fluid may be obtained by reference to C1 hydrocarbon (methane) abundance derived from analysis of the further released gases with abundance in the C1-C36 hydrocarbon distribution.

In the method according to the invention, the analysis of gas released from samples of rock material collected at surface during the drilling of the well or nearby wells are used. Preferably, cuttings are washed to remove drilling mud and air dried prior to storage. Preferably, they are typically collected at about 10 meters intervals and so offer the opportunity to construct "logs" of gas characteristics throughout the drilled section. Ideally, cuttings from the same well may be analyzed, but samples from nearby wells in the same field can be used if such samples are not available. Core chip samples and side wall core samples may also be used for the analysis.

The present invention has been illustrated and described in some of its preferred embodiments, but it is understood that executive variants can be applied to them in practice, without however departing from the scope of the claims.

In relation to the detailed description of the different embodiments of the invention, it will be understood that one or more technical features of one embodiment can be used in combination with one or more technical features of any other embodiment where the transferred use of the one or more technical features would be immediately apparent to a person of ordinary skill in the art to carry out a similar function in a similar way on the other embodiment, without departing from the scope of the claims.

In the preceding discussion of the invention, unless stated to the contrary, the disclosure of alternative values for the upper or lower limit of the permitted range of a parameter, coupled with an indication that one of the values is more highly preferred than the other, is to be construed as an implied statement that each intermediate value of the parameter, lying between the more preferred and the less preferred of the alternatives, is itself preferred to the less preferred value and also to each value lying between the less preferred value and the intermediate value.

The features disclosed in the foregoing description or the following drawings, expressed in their specific forms or in terms of a means for performing a disclosed function, or a method or a process of attaining the disclosed result, as appropriate, may separately, or in any combination of such features be utilized for realizing the invention in diverse forms thereof.

## Claims

1. Method for determining the fluid composition of a reservoir, in particular of a petroleum reservoir, by using at least one rock sample (1) of the reservoir, said method comprising:
- a first analysis (11) of first released gasses, that are released following to the application of a mechanical action, preferably a crushing action, on a first rock sub-sample of said rock sample, so as to determine the molecular quantities of a first hydrocarbons subgroup (12),
- a second analysis (21) of second released gasses and/or a further analysis (61) of further released gasses, that are released following to the application of a mechanical action, preferably a crushing action, on a second rock sub-sample of said rock sample, so as to determine the molecular quantities of a second hydrocarbons subgroup (22) overlapping at least partially with said first hydrocarbons subgroup (12), and/or a further analysis (61) of further released gasses, that are released following to the application of a mechanical action, preferably a crushing action, on a further rock sub-sample of said rock sample, so as to determine the molecular quantities of non-hydrocarbons, preferably carbon dioxide and hydrogen sulphide, and also of at least one hydrocarbon belonging to said first hydrocarbons subgroup (12),
- determining (30) the molecular quantities of an hydrocarbons group (31) comprising said first hydrocarbons subgroup (12) and second hydrocarbons subgroup (22) by using the molecular quantities so determined by means of said first analysis (11) and said second analysis (21) and/or determining (63) the molecular quantities of non-hydrocarbons, preferably carbon dioxide and hydrogen sulphide, by using the molecular quantities so determined by means of said first analysis (11) and by said further analysis (61),
wherein:
- said first hydrocarbons subgroup comprises C1 - C5 hydrocarbons,
- said second hydrocarbons subgroup comprises C4 - C8 hydrocarbons,
- said hydrocarbons group comprises C1 - C8 hydrocarbons,
- the hydrocarbons that are common between the first hydrocarbons subgroup and the second hydrocarbons subgroup comprise C4 and C5 hydrocarbons,
and **characterized in that**:
- pseudo-components of the first hydrocarbons subgroup C1 - C5, comprising total composition of all said hydrocarbons subdivided by carbon number, are derived directly by the integration of the molecular quantities obtained by said first analysis, and
- pseudo-components of the second hydrocarbons subgroup C4 - C8, comprising total composition of all said hydrocarbons subdivided by carbon number, are derived directly by the integration of the molecular quantities obtained by said second analysis.

2. Method according to claim 1, **characterized in that**:
- the first analysis (11) reconstructs a distribution of the molecular quantities of the first hydrocarbons subgroup (12),
- the second analysis (21) reconstructs a distribution of the molecular quantities of the second hydrocarbons subgroup (22),
- said step of determining (30) the molecular quantities of the hydrocarbons group (31) by using the molecular quantities so determined of said first hydrocarbons subgroup and of said second hydrocarbons subgroup comprises the combination of the distribution of the molecular quantities of the first hydrocarbons subgroup (12) with the distribution of the molecular quantities of the second hydrocarbons subgroup (22) by using the molecular quantities of common/overlapping hydrocarbons as reference, so as to obtain a first distribution (31) of the molecular quantities of the hydrocarbons group.

3. Method according to claim 1 or 2, **characterized in that**:
- the first analysis (11) reconstructs a distribution of the molecular quantities of the first hydrocarbons subgroup (12),
- the further analysis (61) reconstructs a distribution of the molecular quantities of at least one hydrocarbon belonging to the first hydrocarbons subgroup (12) and also of non-hydrocarbons, preferably carbon dioxide and hydrogen sulphide,
- said step of determining (63) the molecular quantities of non-hydrocarbons, preferably carbon dioxide and hydrogen sulphide, comprises the combination of the distribution of the molecular quantities of the first hydrocarbons subgroup (12) with the distribution of the molecular quantities of non-hydrocarbons (62), preferably carbon dioxide and hydrogen sulphide, by using as reference the molecular quantities of said at least one hydrocarbon belonging to the first hydrocarbons subgroup (12), so as to obtain an extended distribution of the molecular quantities of the first hydrocarbons subgroup (12) and also of non-hydrocarbons, preferably carbon dioxide and hydrogen sulphide.

4. Method according to one or more of the previous claims, **characterized in that** the method further comprises:
- a third analysis (41) of oil extracted from a third rock sub-sample of said rock sample or of third released fluids, that are released by a chemical treatment or a thermal treatment or by a physical/mechanical action on a third rock sub-sample of said rock sample, so as to determine the molecular quantities of a third hydrocarbons subgroup (42),
- determining (50) the molecular quantities of a wider hydrocarbons group by using the molecular quantities so determined of said first hydrocarbons subgroup, of said second hydrocarbons subgroup and of said third hydrocarbons subgroup.

5. Method according to one or more of the previous claims, **characterized in that** it further comprises the calculation step of the molecular quantities of a fourth hydrocarbons subgroup, said calculation step is based on the molecular quantities so determined of said third hydrocarbons subgroup and/or of said second subgroup.

6. Method according to the previous claim, **characterized in that** the molecular quantities of said fourth hydrocarbons subgroup are calculated by extrapolation using the molecular quantities of said third hydrocarbons subgroup and assuming that in reservoir oil the molar quantity of normal alkanes or pseudo-components with respect to their carbon atom numbers follow an exponential relationship.

7. Method according to one or more of the previous claims, **characterized in that** it also comprises:
- the combination (30) of molecular quantities distribution of the first hydrocarbons subgroup obtained by said first analysis (11) and of said second hydrocarbons subgroup obtained by said second analysis (21), so as to determine a first distribution (31) of the hydrocarbons subdivided by carbon numbers belonging respectively to a first subrange and a second subrange,
- said third analysis (41), so as to determine the distribution (42) of the molecular quantities of said third hydrocarbons subgroup,
- the calculation (45) of molecular quantities of the fourth hydrocarbons subgroup by using the molecular quantities of the third hydrocarbons subgroup, so as to determine a further distribution (46) of the hydrocarbons subdivided by carbon numbers belonging respectively to a third subrange and a fourth subrange, wherein said further distribution (46) having at least one carbon component that is common/overlapped with the first distribution (31),
- the further combination (50) of the first distribution (31) with the further distribution (46) so determined so as to obtain a total distribution (51) of the hydrocarbons subdivided by carbon numbers belonging to the full range.

8. Method according to the previous claim, **characterized in that** said total distribution (51) is normalized to 100%.

9. Method according to one or more of the previous claims, **characterized in that**:
- said third hydrocarbons subgroup comprises C19 - C36 hydrocarbons,
- said fourth hydrocarbons subgroup comprises C8 - C18 hydrocarbons,
- said first distribution (31) comprises the distribution of C1 - C8 hydrocarbons,
- said further distribution (46) comprises the distribution of C8 - C36 hydrocarbons,
- C8 hydrocarbon is the common reference for the first distribution and the further distribution,
- said total hydrocarbons distribution comprises the distribution of C1 - C36 hydrocarbons.

10. Method according to one or more of the previous claims, **characterized in that**:
- said first hydrocarbons subgroup comprises pseudo-components or normal alkanes of C1 - C5 hydrocarbons,
- said second hydrocarbons subgroup comprises pseudo-components or normal alkanes of C4 - C8 hydrocarbons,
- said third hydrocarbons subgroup comprises pseudo-components or normal alkanes of C19 - C36 hydrocarbons,
- said fourth hydrocarbons subgroup comprises pseudo-components or normal alkanes of C8 - C18 hydrocarbons,
- said first distribution (31) comprises the distribution of pseudo-components or normal alkanes of C1 - C8 hydrocarbons,
- said further distribution (46) comprises the distribution of pseudo-components or normal alkanes of C8 - C36 hydrocarbons,
- said total hydrocarbons distribution comprises the distribution of pseudo-components or normal alkanes of C1 - C36 hydrocarbons,
wherein pseudo-components comprise total composition of hydrocarbons subdivided by the corresponding carbon numbers.

11. Method according to one or more of the previous claims, **characterized in that** the rock sample comprise drill cuttings, side wall core chips and/or conventional core chips.

12. Method according to one or more of the previous claims, **characterized in that** it comprises the following steps of:
a) mechanically crushing (10) a first sub-sample of a rock sample of the reservoir to release the first gasses,
b) performing said first analysis (11) of said first released gasses, preferably via gas chromatography with detector, to determine the distribution (12) of molecular quantities of C1-C5 hydrocarbons,
c) repeating step a) on a second sub-sample of a rock sample of the reservoir to release the second gasses, and performing said second analysis (21) of said second gasses, preferably via gas chromatography with detector, using different temperature and chromatographic conditions in respect to the first analysis (11) to determine the distribution (22) of molecular quantities of C4-C8 hydrocarbons,
d) combining (30) the molecular distributions obtained in steps b) and c) using as common reference the relative quantities of C4 - C5 hydrocarbons to construct a first distribution (31) of relative molecular quantities of C1 - C8 hydrocarbons,
e) extracting oil from a third sub-sample of a rock sample of the reservoir and analyzing (41) the oil so extracted,
f) integrating all normal alkanes in the oil chromatogram obtained in step e),
g) determining the distribution (42) of the molecular quantities of C19 - C36 normal alkanes,
h) calculating (45) an exponential curve fitting through the C19 - C36 normal alkanes as determined in step g),
i) extrapolating the molecular quantities of normal alkanes C8 - C18 using the exponential equation obtained in step h) so as to obtain a further distribution (46) with the molecular quantities of C8 - C36 hydrocarbons,
j) further combining (50) the further distribution of C8 - C36 hydrocarbons obtained in step i) and the first distribution of C1-C8 hydrocarbons obtained in step d) by using the quantity of C8 hydrocarbon that is a common reference for both distributions, so as to determine a full distribution (51) of C1-C36 hydrocarbons,
k) re-normalizing to 100% the full distribution (51) of C1-C36 hydrocarbons of step j).

13. Method according to the previous claims, **characterized in that** it further comprises the following steps for the combination of the two distributions of hydrocarbons with non-hydrocarbons:
l) repeating step a) on a further sub-sample of a rock sample to release further gasses and performing a further analysis (61) of said further released gasses, preferably via gas chromatography with thermal conductivity detector, using different temperature and chromatographic conditions in respect to the first analysis (11) in order to determine the molecular quantities of C1 hydrocarbon and also of carbon dioxide and hydrogen sulphide as non-hydrocarbon gases,
m) combining (63) the molecular distributions obtained in steps k) and l) using as common/overlapping reference the relative quantities of C1 hydrocarbon to construct a combined distribution (64) comprising the full distribution (51) of C1-C36 hydrocarbons and also of carbon dioxide and hydrogen sulphide as non-hydrocarbon gases,
n) re-normalizing to 100% said combined distribution (64) of step m).

## Patentansprüche

1. Verfahren zur Bestimmung der Flüssigkeitszusammensetzung eines Reservoirs, insbesondere eines Erdölreservoirs, unter Verwendung mindestens einer Gesteinsprobe (1) des Reservoirs, wobei das Verfahren Folgendes umfasst:
- eine erste Analyse (11) von ersten freigesetzten Gasen, die infolge der Ausübung einer mechanischen Kraft, vorzugsweise einer Zerkleinerungskraft, auf eine erste Gesteinsunterprobe der Gesteinsprobe freigesetzt werden, um die Molekülmengen einer ersten Untergruppe von Kohlenwasserstoffen (12) zu bestimmen,
- eine zweite Analyse (21) von zweiten freigesetzten Gasen und/oder eine weitere Analyse (61) von weiteren freigesetzten Gasen, die infolge der Ausübung einer mechanischen Kraft, vorzugsweise einer Zerkleinerungskraft, auf eine zweite Gesteinsunterprobe der Gesteinsprobe freigesetzt werden, um die Molekülmengen einer zweiten Untergruppe von Kohlenwasserstoffen (22) zu bestimmen, die zumindest teilweise die erste Untergruppe von Kohlenwasserstoffen (12) überlappt, und/oder eine weitere Analyse (61) weiterer freigesetzter Gase, die infolge der Ausübung einer mechanischen Kraft, vorzugsweise einer Zerkleinerungskraft, auf eine weitere Gesteinsunterprobe der Gesteinsprobe freigesetzt werden, um die Molekülmengen von Nicht-Kohlenwasserstoffen, vorzugsweise Kohlendioxid und Schwefelwasserstoff, sowie mindestens eines Kohlenwasserstoffs, der zu der ersten Untergruppe von Kohlenwasserstoffen (12) gehört, zu bestimmen,
- Bestimmen (30) der Molekülmengen einer Gruppe von Kohlenwasserstoffen (31), die die erste Untergruppe von Kohlenwasserstoffen (12) und die zweite Untergruppe von Kohlenwasserstoffen (22) umfasst, unter Verwendung der Molekülmengen, die mittels der ersten Analyse (11) und der zweiten Analyse (21) so bestimmt wurden und/oder Bestimmen (63) der Molekülmengen von Nicht-Kohlenwasserstoffen, vorzugsweise Kohlendioxid und Schwefelwasserstoff, unter Verwendung der Molekülmengen, die mittels der ersten Analyse (11) und der weiteren Analyse (61) so bestimmt wurden,
wobei:
- die erste Untergruppe von Kohlenwasserstoffen C1 - C5-Kohlenwasserstoffe umfasst,
- die zweite Untergruppe von Kohlenwasserstoffen C4 - C8-Kohlenwasserstoffe umfasst,
- die Gruppe von Kohlenwasserstoffen C1 - C8-Kohlenwasserstoffe umfasst,
- die Kohlenwasserstoffe, die zwischen der ersten Untergruppe von Kohlenwasserstoffen und der zweiten Untergruppe von Kohlenwasserstoffen gemeinsam sind, C4- und C5-Kohlenwasserstoffe umfassen,
und **dadurch gekennzeichnet, dass**:
- Pseudo-Komponenten der ersten Untergruppe von Kohlenwasserstoffen C1-C5, die die Gesamtzusammensetzung aller Kohlenwasserstoffe, unterteilt nach der Kohlenstoffzahl, umfassen, direkt durch die Integration der durch die erste Analyse erhaltenen Molekülmengen abgeleitet werden, und
- Pseudo-Komponenten der zweiten Untergruppe von Kohlenwasserstoffen C4-C8, die die Gesamtzusammensetzung aller Kohlenwasserstoffe, unterteilt nach der Kohlenstoffzahl, umfassen, direkt durch die Integration der durch die zweite Analyse erhaltenen Molekülmengen abgeleitet werden.

2. Verfahren nach Anspruch 1, **dadurch gekennzeichnet, dass**:
- die erste Analyse (11) eine Verteilung der Molekülmengen der ersten Untergruppe von Kohlenwasserstoffen (12) rekonstruiert,
- die zweite Analyse (21) eine Verteilung der Molekülmengen der zweiten Untergruppe von Kohlenwasserstoffen (22) rekonstruiert,
- der Schritt des Bestimmens (30) der Molekülmengen der Gruppe von Kohlenwasserstoffen (31) unter Verwendung der so bestimmten Molekülmengen der ersten Untergruppe von Kohlenwasserstoffen und der zweiten Untergruppe von Kohlenwasserstoffen die Kombination der Verteilung der Molekülmengen der ersten Untergruppe von Kohlenwasserstoffen (12) mit der Verteilung der Molekülmengen der zweiten Untergruppe von Kohlenwasserstoffen (22) unter Verwendung der Molekülmengen gemeinsamer/überlappender Kohlenwasserstoffe als Referenz umfasst, um eine erste Verteilung (31) der Molekülmengen der Gruppe von Kohlenwasserstoffen zu erhalten.

3. Verfahren nach Anspruch 1 oder 2, **dadurch gekennzeichnet, dass**:
- die erste Analyse (11) eine Verteilung der Molekülmengen der ersten Untergruppe von Kohlenwasserstoffen (12) rekonstruiert,
- die weitere Analyse (61) eine Verteilung der Molekülmengen mindestens eines zur ersten Untergruppe von Kohlenwasserstoffen (12) gehörenden Kohlenwasserstoffs sowie von Nicht-Kohlenwasserstoffen, vorzugsweise Kohlendioxid und Schwefelwasserstoff, rekonstruiert,
- der Schritt des Bestimmens (63) der Molekülmengen von Nicht-Kohlenwasserstoffen, vorzugsweise Kohlendioxid und Schwefelwasserstoff, die Kombination der Verteilung der Molekülmengen der ersten Untergruppe von Kohlenwasserstoffen (12) mit der Verteilung der Molekülmengen von Nicht-Kohlenwasserstoffen (62), vorzugsweise Kohlendioxid und Schwefelwasserstoff, umfasst, indem die Molekülmengen des mindestens einen Kohlenwasserstoffs, der zur ersten Untergruppe von Kohlenwasserstoffen (12) gehört, als Referenz verwendet werden, um eine erweiterte Verteilung der Molekülmengen der ersten Untergruppe von Kohlenwasserstoffen (12), sowie von Nicht-Kohlenwasserstoffen, vorzugsweise Kohlendioxid und Schwefelwasserstoff, zu erhalten.

4. Verfahren nach einem oder mehreren der vorhergehenden Ansprüche, **dadurch gekennzeichnet, dass** das Verfahren ferner Folgendes umfasst:
- eine dritte Analyse (41) von Öl, das aus einer dritten Gesteinsunterprobe der Gesteinsprobe gewonnen wird oder von dritten freigesetzten Flüssigkeiten, die durch eine chemische Behandlung oder eine thermische Behandlung oder durch eine physikalische/mechanische Kraft auf eine dritte Gesteinsunterprobe der Gesteinsprobe freigesetzt werden, um die Molekülmengen einer dritten Untergruppe von Kohlenwasserstoffen (42) zu bestimmen,
- Bestimmen (50) der Molekülmengen einer breiteren Gruppe von Kohlenwasserstoffen unter Verwendung der so bestimmten Molekülmengen der ersten Untergruppe von Kohlenwasserstoffen, der zweiten Untergruppe von Kohlenwasserstoffen und der dritten Untergruppe von Kohlenwasserstoffen.

5. Verfahren nach einem oder mehreren der vorhergehenden Ansprüche, **dadurch gekennzeichnet, dass** es ferner den Berechnungsschritt der Molekülmengen einer vierten Untergruppe von Kohlenwasserstoffen umfasst, wobei der Berechnungsschritt auf den so bestimmten Molekülmengen der dritten Untergruppe von Kohlenwasserstoffen und/oder der zweiten Untergruppe basiert.

6. Verfahren nach dem vorhergehenden Anspruch, **dadurch gekennzeichnet, dass** die Molekülmengen der vierten Untergruppe von Kohlenwasserstoffen durch Extrapolation unter Verwendung der Molekülmengen der dritten Untergruppe von Kohlenwasserstoffen und unter der Annahme berechnet werden, dass im Reservoiröl die Molmenge an normalen Alkanen oder Pseudo-Komponenten in Bezug auf ihre Kohlenstoffatomzahlen einer exponentiellen Beziehung folgt.

7. Verfahren nach einem oder mehreren der vorhergehenden Ansprüche, **dadurch gekennzeichnet, dass** es auch umfasst:
- die Kombination (30) der Molekularmengenverteilung der ersten Untergruppe von Kohlenwasserstoffen, die durch die erste Analyse (11) erhalten wurde, und der zweiten Untergruppe von Kohlenwasserstoffen, die durch die zweite Analyse (21) erhalten wurde, um eine erste Verteilung (31) der Kohlenwasserstoffe zu bestimmen, unterteilt nach den Kohlenstoffzahlen, die jeweils zu einem ersten Unterbereich und einem zweiten Unterbereich gehören,
- die dritte Analyse (41), um die Verteilung (42) der Molekülmengen der dritten Untergruppe von Kohlenwasserstoffen zu bestimmen,
- die Berechnung (45) von Molekülmengen der vierten Untergruppe von Kohlenwasserstoffen unter Verwendung der Molekülmengen der dritten Untergruppe von Kohlenwasserstoffen, um eine weitere Verteilung (46) der Kohlenwasserstoffe zu bestimmen, unterteilt nach den Kohlenstoffzahlen, die jeweils zu einem dritten Unterbereich und einem vierten Unterbereich gehören, wobei die weitere Verteilung (46) mindestens eine Kohlenstoffkomponente aufweist, die mit der ersten Verteilung (31) gemeinsam/überlappt ist,
- die weitere Kombination (50) der ersten Verteilung (31) mit der so bestimmten weiteren Verteilung (46), um eine Gesamtverteilung (51) der Kohlenwasserstoffe zu erhalten, unterteilt nach den Kohlenstoffzahlen, die dem vollständigen Bereich gehören.

8. Verfahren nach dem vorhergehenden Anspruch, **dadurch gekennzeichnet, dass** die Gesamtverteilung (51) auf 100 % normalisiert wird.

9. Verfahren nach einem oder mehreren der vorhergehenden Ansprüche, **dadurch gekennzeichnet, dass**:
- die dritte Untergruppe von Kohlenwasserstoffen C19 - C36-Kohlenwasserstoffe umfasst,
- die vierte Untergruppe von Kohlenwasserstoffen C8 - C18-Kohlenwasserstoffe umfasst,
- die erste Verteilung (31) die Verteilung von C1 - C8-Kohlenwasserstoffen umfasst,
- die weitere Verteilung (46) die Verteilung von C8 - C36-Kohlenwasserstoffen umfasst,
- C8-Kohlenwasserstoff als die gemeinsame Referenz für die erste Verteilung und die weitere Verteilung dient,
- die gesamte Kohlenwasserstoffverteilung die Verteilung von C1 - C36-Kohlenwasserstoffen umfasst.

10. Verfahren nach einem oder mehreren der vorhergehenden Ansprüche, **dadurch gekennzeichnet, dass**:
- die erste Untergruppe von Kohlenwasserstoffen Pseudo-Komponenten oder normale Alkane von C1 - C5-Kohlenwasserstoffen umfasst,
- die zweite Untergruppe von Kohlenwasserstoffen Pseudo-Komponenten oder normale Alkane von C4 - C8-Kohlenwasserstoffen umfasst,
- die dritte Untergruppe von Kohlenwasserstoffen Pseudo-Komponenten oder normale Alkane von C19 - C36-Kohlenwasserstoffen umfasst,
- die vierte Untergruppe von Kohlenwasserstoffen Pseudo-Komponenten oder normale Alkane von C8 - C18-Kohlenwasserstoffen umfasst,
- die erste Verteilung (31) die Verteilung von Pseudo-Komponenten oder normalen Alkanen von C1 - C8-Kohlenwasserstoffen umfasst,
- die weitere Verteilung (46) die Verteilung von Pseudo-Komponenten oder normalen Alkanen von C8 - C36-Kohlenwasserstoffen umfasst,
- die Gesamtkohlenwasserstoffverteilung die Verteilung von Pseudo-Komponenten oder normalen Alkanen von C1 - C36-Kohlenwasserstoffen umfasst,
wobei Pseudo-Komponenten die Gesamtzusammensetzung von Kohlenwasserstoffen umfassen, die nach den entsprechenden Kohlenstoffzahlen unterteilt sind.

11. Verfahren nach einem oder mehreren der vorhergehenden Ansprüche, **dadurch gekennzeichnet, dass** die Gesteinsprobe Bohrklein, Seitenwandkernspäne und/oder konventionelle Kernspäne umfasst.

12. Verfahren nach einem oder mehreren der vorhergehenden Ansprüche, **dadurch gekennzeichnet, dass** es die folgenden Schritte umfasst:
a) mechanisches Zerkleinern (10) einer ersten Unterprobe einer Gesteinsprobe des Reservoirs, um die ersten Gase freizusetzen,
b) Durchführen der ersten Analyse (11) der ersten freigesetzten Gase, vorzugsweise mittels Gaschromatographie mit Detektor, zur Bestimmung der Verteilung (12) der Molekülmengen von C1-C5-Kohlenwasserstoffen,
c) Wiederholen von Schritt a) an einer zweiten Unterprobe einer Gesteinsprobe des Reservoirs, um die zweiten Gase freizusetzen, und Durchführen der zweiten Analyse (21) der zweiten Gase, vorzugsweise mittels Gaschromatographie mit Detektor, unter Verwendung unterschiedlicher Temperatur und chromatographischer Bedingungen als bei der ersten Analyse (11), um die Verteilung (22) von Molekülmengen von C4-C8-Kohlenwasserstoffen zu bestimmen,
d) Kombinieren (30) der in den Schritten b) und c) erhaltenen Molekülverteilungen unter Verwendung der relativen Mengen von C4- bis C5-Kohlenwasserstoffen als gemeinsame Referenz, um eine erste Verteilung (31) der relativen Molekülmengen von C1- bis C8-Kohlenwasserstoffen zu konstruieren,
e) Gewinnen von Öl aus einer dritten Unterprobe einer Gesteinsprobe des Reservoirs und Analysieren (41) des so gewonnenen Öls,
f) Einbeziehen aller normalen Alkane in das in Schritt e) erhaltene Ölchromatogramm,
g) Bestimmen der Verteilung (42) der Molekülmengen von C19 - C36- normalen Alkanen,
h) Berechnen (45) einer exponentiellen Kurvenanpassung durch die C19 - C36-normalen Alkane, wie in Schritt g) bestimmt,
i) Extrapolieren der Molekülmengen von normalen Alkanen C8 - C18 unter Verwendung der in Schritt h) erhaltenen Exponentialgleichung, um eine weitere Verteilung (46) mit den Molekülmengen von C8 - C36-Kohlenwasserstoffen zu erhalten,
j) weiteres Kombinieren (50) der in Schritt i) erhaltenen weiteren Verteilung von C8-C36-Kohlenwasserstoffen und der in Schritt d) erhaltenen ersten Verteilung von C1-C8-Kohlenwasserstoffen unter Verwendung der Menge von C8-Kohlenwasserstoffen, die als eine gemeinsame Referenz für beide Verteilungen dient, um eine vollständige Verteilung (51) von C1-C36-Kohlenwasserstoffen zu bestimmen,
k) Wieder-Normalisieren der vollständigen Verteilung (51) der C1-C36-Kohlenwasserstoffe aus Schritt j) auf 100 %.

13. Verfahren nach den vorhergehenden Ansprüchen, **dadurch gekennzeichnet, dass** es ferner die folgenden Schritte für die Kombination der zwei Verteilungen von Kohlenwasserstoffen mit Nicht-Kohlenwasserstoffen umfasst:
I) Wiederholen des Schritts a) an einer weiteren Unterprobe einer Gesteinsprobe, um weitere Gase freizusetzen, und Durchführen einer weiteren Analyse (61) dieser weiter freigesetzten Gase, vorzugsweise mittels Gaschromatographie mit einem Wärmeleitfähigkeitsdetektor, unter Verwendung unterschiedlicher Temperatur und chromatographischer Bedingungen als bei der ersten Analyse (11), um die Molekülmengen von C1-Kohlenwasserstoff sowie von Kohlendioxid und Schwefelwasserstoff als Nicht-Kohlenwasserstoffgase zu bestimmen,
m) Kombinieren (63) der in den Schritten k) und I) erhaltenen Molekülverteilungen unter Verwendung der relativen Mengen von C1-Kohlenwasserstoffen als gemeinsame/überlappende Referenz, um eine kombinierte Verteilung (64) zu konstruieren, die die vollständige Verteilung (51) von C1-C36-Kohlenwasserstoffen sowie von Kohlendioxid und Schwefelwasserstoff als Nicht-Kohlenwasserstoff-Gase umfasst,
n) Wieder-Normalisieren der in Schritt m) kombinierten Verteilung (64) auf 100 %.

## Revendications

1. Procédé permettant de déterminer la composition des fluides d'un réservoir, en particulier d'un réservoir de pétrole, à l'aide d'au moins un échantillon de roche (1) du réservoir, ledit procédé comprenant :
- une première analyse (11) de premiers gaz libérés, étant libérés à la suite de l'application d'une action mécanique, de préférence une action de fragmentation, sur un premier sous-échantillon de roche dudit échantillon de roche, afin de déterminer les quantités moléculaires d'un premier sous-groupe d'hydrocarbures (12),
- une deuxième analyse (21) de seconds gaz libérés et/ou une analyse supplémentaire (61) de gaz libérés supplémentaires, étant libérés à la suite de l'application d'une action mécanique, de préférence une action de fragmentation, sur un deuxième sous-échantillon de roche dudit échantillon de roche, de manière à déterminer les quantités moléculaires d'un deuxième sous-groupe d'hydrocarbures (22) se chevauchant au moins partiellement avec ledit premier sous-groupe d'hydrocarbures (12), et/ou une analyse supplémentaire (61) de gaz libérés supplémentaires, étant libérés à la suite de l'application d'une action mécanique, de préférence une action de fragmentation, sur un sous-échantillon de roche supplémentaire dudit échantillon de roche, afin de déterminer les quantités moléculaires de non-hydrocarbures, de préférence le dioxyde de carbone et le sulfure d'hydrogène, ainsi que d'au moins un hydrocarbure appartenant audit premier sous-groupe d'hydrocarbures (12),
- la détermination (30) des quantités moléculaires d'un groupe d'hydrocarbures (31) comprenant lesdits premier sous-groupe d'hydrocarbures (12) et deuxième sous-groupe d'hydrocarbures (22) en utilisant les quantités moléculaires ainsi déterminées au moyen de ladite première analyse (11) et de ladite deuxième analyse (21) et/ou la détermination (63) des quantités moléculaires de non-hydrocarbures, de préférence le dioxyde de carbone et le sulfure d'hydrogène, en utilisant les quantités moléculaires ainsi déterminées au moyen de ladite première analyse (11) et par ladite analyse supplémentaire (61),
dans lequel :
- ledit premier sous-groupe d'hydrocarbures comprend des hydrocarbures C1 - C5,
- ledit deuxième sous-groupe d'hydrocarbures comprend des hydrocarbures C4 - C8,
- ledit groupe d'hydrocarbures comprend des hydrocarbures C1 - C8,
- les hydrocarbures étant communs au premier sous-groupe d'hydrocarbures et au deuxième sous-groupe d'hydrocarbures comprennent des hydrocarbures C4 et C5,
et **caractérisé en ce que** :
- des pseudo-composants du premier sous-groupe d'hydrocarbures C1 - C5, comprenant la composition totale de tous lesdits hydrocarbures subdivisés en fonction du nombre d'atomes de carbone, sont directement dérivés de l'intégration des quantités moléculaires obtenues par ladite première analyse, et
- des pseudo-composants du deuxième sous-groupe d'hydrocarbures C4 - C8, comprenant la composition totale de tous lesdits hydrocarbures subdivisés en fonction du nombre d'atomes de carbone, sont directement dérivés de l'intégration des quantités moléculaires obtenues par ladite deuxième analyse.

2. Procédé selon la revendication 1, **caractérisé en ce que** :
- la première analyse (11) reconstitue une répartition des quantités moléculaires du premier sous-groupe d'hydrocarbures (12),
- la deuxième analyse (21) reconstitue une répartition des quantités moléculaires du deuxième sous-groupe d'hydrocarbures (22),
- ladite étape de détermination (30) des quantités moléculaires du groupe d'hydrocarbures (31) à l'aide des quantités moléculaires ainsi déterminées dudit premier sous-groupe d'hydrocarbures et dudit deuxième sous-groupe d'hydrocarbures comprend la combinaison de la répartition des quantités moléculaires du premier sous-groupe d'hydrocarbures (12) avec la répartition des quantités moléculaires du deuxième sous-groupe d'hydrocarbures (22) en utilisant comme référence les quantités moléculaires d'hydrocarbures communs/se chevauchant, de manière à obtenir une première répartition (31) des quantités moléculaires du groupe d'hydrocarbures.

3. Procédé selon la revendication 1 ou 2, **caractérisé en ce que** :
- la première analyse (11) reconstitue une répartition des quantités moléculaires du premier sous-groupe d'hydrocarbures (12),
- l'analyse supplémentaire (61) reconstitue une répartition des quantités moléculaires d'au moins un hydrocarbure appartenant au premier sous-groupe d'hydrocarbures (12), ainsi que de non-hydrocarbures, de préférence le dioxyde de carbone et le sulfure d'hydrogène,
- ladite étape de détermination (63) des quantités moléculaires de non-hydrocarbures, de préférence le dioxyde de carbone et le sulfure d'hydrogène, comprend la combinaison de la répartition des quantités moléculaires du premier sous-groupe d'hydrocarbures (12) avec la répartition des quantités moléculaires des non-hydrocarbures (62), de préférence le dioxyde de carbone et le sulfure d'hydrogène, en utilisant comme référence les quantités moléculaires dudit au moins un hydrocarbure appartenant au premier sous-groupe d'hydrocarbures (12), de manière à obtenir une répartition étendue des quantités moléculaires du premier sous-groupe d'hydrocarbures (12) ainsi que des non-hydrocarbures, de préférence le dioxyde de carbone et le sulfure d'hydrogène.

4. Procédé selon l'une ou plusieurs des revendications précédentes, **caractérisé en ce que** le procédé comprend en outre :
- une troisième analyse (41) de l'huile extraite d'un troisième sous-échantillon de roche dudit échantillon de roche ou de troisièmes fluides libérés, qui sont libérés par un traitement chimique ou un traitement thermique ou par une action physique/mécanique sur un troisième sous-échantillon de roche dudit échantillon de roche, de manière à déterminer les quantités moléculaires d'un troisième sous-groupe d'hydrocarbures (42),
- la détermination (50) des quantités moléculaires d'un groupe plus large d'hydrocarbures en utilisant les quantités moléculaires ainsi déterminées dudit premier sous-groupe d'hydrocarbures, dudit deuxième sous-groupe d'hydrocarbures et dudit troisième sous-groupe d'hydrocarbures.

5. Procédé selon l'une ou plusieurs des revendications précédentes, **caractérisé en ce qu'**il comprend en outre l'étape consistant à calculer les quantités moléculaires d'un quatrième sous-groupe d'hydrocarbures, ladite étape de calcul est basée sur les quantités moléculaires ainsi déterminées dudit troisième sous-groupe d'hydrocarbures et/ou dudit deuxième sous-groupe.

6. Procédé selon la revendication précédente, **caractérisé en ce que** les quantités moléculaires dudit quatrième sous-groupe d'hydrocarbures sont calculées par extrapolation en utilisant les quantités moléculaires dudit troisième sous-groupe d'hydrocarbures, en supposant que, dans l'huile de réservoir, la quantité molaire des alcanes normaux ou des pseudo-composants, par rapport à leur nombre d'atomes de carbone, suit une relation exponentielle.

7. Procédé selon l'une ou plusieurs des revendications précédentes, **caractérisé en ce qu'**il comprend en outre :
- la combinaison (30) de la répartition des quantités moléculaires du premier sous-groupe d'hydrocarbures obtenu par ladite première analyse (11) et de celle dudit deuxième sous-groupe d'hydrocarbures obtenu par ladite deuxième analyse (21), afin de déterminer une première répartition (31) des hydrocarbures subdivisés en fonction du nombre d'atomes de carbone appartenant respectivement à une première sous-plage et à une deuxième sous-plage,
- ladite troisième analyse (41), afin de déterminer la répartition (42) des quantités moléculaires dudit troisième sous-groupe d'hydrocarbures,
- le calcul (45) des quantités moléculaires du quatrième sous-groupe d'hydrocarbures à l'aide des quantités moléculaires du troisième sous-groupe d'hydrocarbures, afin de déterminer une répartition supplémentaire (46) des hydrocarbures subdivisés en fonction du nombre d'atomes de carbone appartenant respectivement à une troisième sous-plage et à une quatrième sous-plage, ladite répartition supplémentaire (46) comportant au moins un composant carboné étant commun/se chevauchant avec la première répartition (31),
- la combinaison supplémentaire (50) de la première répartition (31) avec la répartition supplémentaire (46) ainsi déterminée, de manière à obtenir une répartition totale (51) des hydrocarbures subdivisés en fonction du nombre d'atomes de carbone appartenant à la plage entière.

8. Procédé selon la revendication précédente, **caractérisé en ce que** ladite répartition totale (51) est normalisée à 100 %.

9. Procédé selon l'une ou plusieurs des revendications précédentes, **caractérisé en ce que** :
- ledit troisième sous-groupe d'hydrocarbures comprend des hydrocarbures C19 - C36,
- ledit quatrième sous-groupe d'hydrocarbures comprend des hydrocarbures C8 - C18,
- ladite première répartition (31) comprend la répartition d'hydrocarbures C1 - C8,
- ladite répartition supplémentaire (46) comprend la répartition d'hydrocarbures C8 - C36,
- L'hydrocarbure C8 est la référence commune pour la première répartition et la répartition supplémentaire,
- ladite répartition des hydrocarbures totaux comprend la répartition des hydrocarbures C1 - C36.

10. Procédé selon l'une ou plusieurs des revendications précédentes, **caractérisé en ce que** :
- ledit premier sous-groupe d'hydrocarbures comprend des pseudo-composants ou des alcanes normaux d'hydrocarbures C1 - C5,
- ledit deuxième sous-groupe d'hydrocarbures comprend des pseudo-composants ou des alcanes normaux d'hydrocarbures C4 - C8,
- ledit troisième sous-groupe d'hydrocarbures comprend des pseudo-composants ou des alcanes normaux d'hydrocarbures C19 - C36,
- ledit quatrième sous-groupe d'hydrocarbures comprend des pseudo-composants ou des alcanes normaux d'hydrocarbures C8 - C18,
- ladite première répartition (31) comprend la répartition de pseudo-composants ou d'alcanes normaux d'hydrocarbures C1 - C8,
- ladite répartition supplémentaire (46) comprend la répartition de pseudo-composants ou d'alcanes normaux d'hydrocarbures C8 - C36,
- ladite répartition des hydrocarbures totaux comprend la répartition des pseudo-composants ou des alcanes normaux d'hydrocarbures C1 - C36,
- les pseudo-composants comprenant la composition totale des hydrocarbures subdivisés en fonction du nombre d'atomes de carbone correspondants.

11. Procédé selon l'une ou plusieurs des revendications précédentes, **caractérisé en ce que** l'échantillon de roche comprend des déblais de forage, des fragments de carotte de parois latérales et/ou des fragments de carotte conventionnels.

12. Procédé selon l'une ou plusieurs des revendications précédentes, **caractérisé en ce qu'**il comprend les étapes suivantes :
a) la fragmentation mécanique (10) d'un premier sous-échantillon d'un échantillon de roche du réservoir afin de libérer les premiers gaz,
b) la réalisation de ladite première analyse (11) desdits premiers gaz libérés, de préférence par chromatographie en phase gazeuse avec détecteur, afin de déterminer la répartition (12) des quantités moléculaires d'hydrocarbures C1-C5,
c) la répétition de l'étape a) sur un deuxième sous-échantillon d'un échantillon de roche du réservoir afin de libérer les seconds gaz, et effectuer ladite deuxième analyse (21) desdits seconds gaz, de préférence par chromatographie en phase gazeuse avec détecteur, en utilisant des conditions de température et de chromatographie différentes de celles de la première analyse (11) afin de déterminer la répartition (22) des quantités moléculaires d'hydrocarbures C4-C8,
d) la combinaison (30) des répartitions moléculaires obtenues aux étapes b) et c) en utilisant comme référence commune les quantités relatives d'hydrocarbures C4 - C5 afin de constituer une première répartition (31) des quantités moléculaires relatives d'hydrocarbures C1 - C8,
e) l'extraction d'huile d'un troisième sous-échantillon d'un échantillon de roche du réservoir et l'analyse (41) de l'huile ainsi extraite,
f) l'intégration de tous les alcanes normaux dans le chromatogramme d'huile obtenu à l'étape e),
g) la détermination de la répartition (42) des quantités moléculaires d'alcanes normaux C19 - C36,
h) le calcul (45) d'une courbe exponentielle s'ajustant aux alcanes normaux C19 - C36, tels que déterminés à l'étape g),
i) l'extrapolation des quantités moléculaires des alcanes normaux C8 - C18 à l'aide de l'équation exponentielle obtenue à l'étape h) afin d'obtenir une répartition supplémentaire (46) avec les quantités moléculaires des hydrocarbures C8 - C36,
j) la combinaison supplémentaire (50) de la répartition supplémentaire des hydrocarbures C8 - C36 obtenue à l'étape i) et la première répartition des hydrocarbures C1 - C8 obtenue à l'étape d) en utilisant la quantité d'hydrocarbure C8 servant de référence commune aux deux répartitions, de manière à déterminer une répartition complète (51) d'hydrocarbures C1-C36,
k) la renormalisation à 100 % de la répartition complète (51) des hydrocarbures C1-C36 de l'étape j).

13. Procédé selon les revendications précédentes, **caractérisé en ce qu'**il comprend en outre les étapes suivantes pour la combinaison des deux répartitions des hydrocarbures et des non-hydrocarbures :
l) la répétition de l'étape a) sur un sous-échantillon supplémentaire d'un échantillon de roche afin de libérer des gaz supplémentaires et effectuer une analyse supplémentaire (61) desdits gaz supplémentaires libérés, de préférence par chromatographie en phase gazeuse avec détecteur à conductivité thermique, en utilisant des conditions de température et de chromatographie différentes de celles de la première analyse (11) afin de déterminer les quantités moléculaires d'hydrocarbures C1 ainsi que de dioxyde de carbone et de sulfure d'hydrogène comme non-hydrocarbures gazeux,
m) la combinaison (63) des répartitions moléculaires obtenues aux étapes k) et l) en utilisant comme référence commune/se superposant les quantités relatives d'hydrocarbures C1 afin de constituer une répartition combinée (64) comprenant la répartition complète (51) des hydrocarbures C1-C36 ainsi que celle du dioxyde de carbone et du sulfure d'hydrogène comme non-hydrocarbures gazeux,
n) la renormalisation à 100 % de ladite répartition combinée (64) de l'étape m).
